# EUROPÄISCHE PATENTSCHRIFT

(11) **EP 0 808 306 B1**
(45) Veröffentlichungstag und Bekanntmachung des Hinweises auf die Patenterteilung: **04.08.1999**
(21) Anmeldenummer: 96904021.1
(22) Anmeldetag: 08.02.1996
(51) Int. Cl.: C07D 213/74, C07D 401/12, C07D 237/20, A61K 31/44, A61K 31/50

(54) **NEUE PYRIDIN- BZW. PYRIDAZINDERIVATE, VERFAHREN ZU DEREN HERSTELLUNG UND DIESE VERBINDUNGEN ENTHALTENDE ARZNEIMITTEL**
NEW DERIVATIVES OF PYRIDINE OR PYRIDAZINE, PROCESS FOR PRODUCTION THEREOF AND MEDICAMENTS CONTAINING THESE COMPOUNDS
NOUVEAUX DERIVES DE PYRIDINE OU DE PYRIDAZINE, LEURS PROCEDES DE PRODUCTION, ET MEDICAMENTS CONTENANT CES COMPOSES

(30) Priorität: 10.02.1995 DE 19504367
(43) Veröffentlichungstag der Anmeldung: 26.11.1997
(73) Patentinhaber: Roche Diagnostics GmbH, 68298 Mannheim (DE)
(72) Erfinder: TSAKLAKIDIS, Christos, D-69469 Weinheim (DE); MERTENS, Alfred, D-69198 Schriesheim (DE); ZIMMERMANN, Gerd, D-68309 Mannheim (DE); SCHÄFER, Wolfgang, D-68199 Mannheim (DE); DÖRGE, Liesel, D-68259 Mannheim (DE)
(86) Internationale Anmeldenummer: EP9600523
(87) Internationale Veröffentlichungsnummer: WO9624586

(56) Entgegenhaltungen:
- WO-A-94/22835

## Beschreibung

Es ist bekannt, daß Verbindungen, die eine basische und eine saure Gruppe tragen, in der Lage sind, die Blutplättchen-Aggregation zu hemmen, wenn die basische und die saure Gruppe in den Verbindungen einen ganz bestimmten Abstand zueinander einnehmen (Drugs of the Future 19(8), 757 (1994). In den Patentschriften WO 93/14077, EP0537980 A1, EP0542363 A2, WO 94/22834 und WO 94/22835 sind Verbindungen mit antiaggregatorischer Wirkung an den Blutplättchen beschrieben.

Die vorliegende Erfindung betrifft neue Pyridin- bzw. Pyridazinderivate, Verfahren zu deren Herstellung sowie Arzneimittel, die diese Substanzen enthalten.

Es wurde nun überaschenderweise gefunden, daß Pyridin- bzw. Pyridazinderivate, die noch eine Carbonsäurefunktion tragen, effektiv die Aggregation der Blutplättchen hemmen und damit zur breiten Behandlung von Krankheiten eingesetzt werden können, die auf thromboembolische Ereignisse zurückzuführen sind, wie Schlaganfall. Myocardinfarkt oder arterielle Verschlußkrankheiten, sowie Entzündungen, Osteoporose oder Tumorerkrankungen

Gegenstand der vorliegenden Erfindung sind Verbindungen der allgemeinen Formel I, 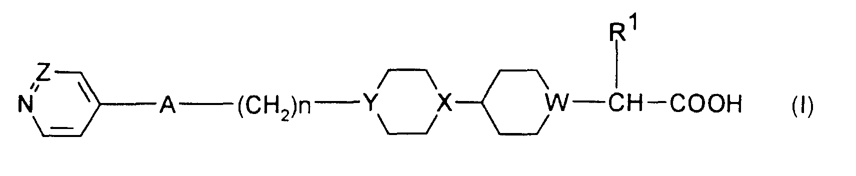 in der
- R¹: Wasserstoff, niederes Alkyl, niederes Alkenyl, Cycloalkyl, Cycloalkenyl, ein gegebenenfalls substituiertes mono- oder bicyclisches Aryl, ein gegebenenfalls substituiertes Hetaryl, ein gegebenenfalls substituiertes Arylalkyl oder eine der Gruppen

-OR², -NR³R⁴

bedeutet,
- W: Stickstoff oder 〉̶CR⁵ bedeutet,
- X, Y, Z: unabhängig voneinander Stickstoff oder die Gruppe 〉̶CH bedeuten, und für den Fall, daß W die Gruppe 〉̶CR⁵ und X die Gruppe 〉̶CH bedeuten. Y nicht die 〉̶CH-Gruppe sein darf,
- A: Sauerstoff, 〉NR² oder 〉N-P bedeutet,
- n: 0-5 bedeutet,
- P: eine Schutzgruppe für Amine wie Acetyl, tert.-Butyloxycarbonyl oder Benzyloxycarbonyl bedeutet,
- R²: Wasserstoff, niederes Alkyl oder Arylalkyl bedeutet,
- R³, R⁴: unabhängig voneinander Wasserstoff, oder niederes Alkyl bedeuten oder zusammen mit dem Stickstoffatom, an das sie gebunden sind, einen fünf- bis sechsgliedrigen heterocyclischen Ring bilden,
- R⁵: Wasserstoff oder eine Gruppe -OR² bedeutet,
sowie deren pharmakologisch unbedenklichen Salze.

Niederes Alkyl soll in allen Fällen eine geradkettige oder verzweigte C₁-C₆-Alkylgruppe wie z.B. Methyl, Ethyl, Propyl, Isopropyl, Butyl, Isobutyl, Pentyl oder Hexyl, insbesondere Methyl, Ethyl, Propyl, Isobutyl und Pentyl darstellen.

Niederes Alkenyl bedeutet ungesättigte Reste mit 3-6 Kohlenstoffatomen wie z.B. Allyl, But-2-enyl, Hexa-2,4-dienyl, vor allem Allyl.

Cycloalkyl bedeutet einen gegebenenfalls substituierten 3-7-gliedrigen Ring, wie den Cyclopropyl-, Cyclobutyl-, Cyclopentyl-, Cyclohexyl- oder den Cycloheptylring, insbesondere den Cyclopropyl, Cyclopentyl- und Cyclohexylring. Diese Cycloalkylreste können ein- oder zweifach durch C₁-C₆-Alkylgruppe, vorzugsweise die Methyl-, Ethyl- oder Isopropylgruppe, sowie durch Hydroxy-, Methoxy-, Benzyloxy-, Amino-, Methylamino-, Dimethylamino-, Benzylaminogruppen oder durch Chlor oder Brom substituiert sein.

Cycloalkenyl bedeutet einen gegebenenfalls substituierten Cyclopentenyl-, Cyclohexenyl- oder Cycloheptenylring. Diese Ringe können ein- oder zweifach durch C₁-C₆-Alkylgruppe, vorzugsweise die Methyl, Ethyl oder Isopropylgruppe, sowie Chlor, Brom oder Hydroxy-, Methoxy-, Benzyloxy-, Amino-, Methylamino-, Dimethylamino- oder Benzylaminogruppen substituiert sein.

Falls die Reste R³ und R⁴ zusammen mit dem Stickstoffatom an das sie gebunden sind einen heterocyclischen Ring bilden, handelt es sich um einen gesättigten oder ungesättigten 5 - 6-gliedrigen Ring, wie den Pyrrolidin-, Piperidin-, Morpholin-, Pyrrolinring.

Die carbocyclischen und heterocyclischen Ringe können gegebenenfalls ein oder zweifach durch C₁-C₆-Alkylgruppen, vorzugsweise die Methyl-, Ethyl-, oder Isopropylgruppe, sowie durch Chlor, Brom oder Hydroxy-, Methoxy- Benzyloxy-, Amino-, Methylamino-, Dimethylamino- oder Benzylaminogruppen substituiert sein.

Aryl bedeutet in der Regel den gegebenenfalls ein- oder mehrfach substituierten Phenylrest.

Hetaryl bedeutet in der Regel ein- oder mehrfach substituierten Pyridin-, Pyridazin-, Pyrrol-, Thiopnen-, Furan- oder Imidazolring.

Bicyclisches Aryl bedeutet in der Regel einen gegebenenfalls ein- oder mehrfach substituierten Indan- oder Naphthalinrest, vorzugsweise den Naphthalinrest.

Aryl-, bicyclische Aryl- und Hetarylreste können gegebenenfalls ein- oder mehrfach durch C₁-C₆-Alkylgruppen, vorzugsweise die Methyl-, Ethyl- oder Isopropylgruppe, sowie durch Chlor, Brom, Fluor, oder Hydroxy-, Alkoxy wie z.B. Methoxy-, Benzyloxy-, Acetyloxy-, Carboxy-, Ethoxycarbonyl-, Aminocarbonyl-, Methylaminocarbonyl-, Dimethylaminocarbonyl-, Cyano-, Amino-, Methylamino-, Dimethylamino-, Benzylamino-, Acetylamino-, Benzoylamino- und Amidingruppen substituiert sein.

Arylalkyl bedeutet in der Regel einen unsubstituierten oder ein- oder mehrfach substituierten Benzyl-, Phenethyl-, Phenylpropyl-, Phenylbutyl- oder Phenylpentylrest, vorzugsweise einen Benzyl-, Phenethyl- oder Phenylpentylrest. Als Substituenten kommen C₁-C₆-Alkylreste vorzugsweise Methyl-, Ethyl- oder Isopropyl, sowie Chlor, Brom, Fluor, oder Hydroxy-, Methoxy-, Benzyloxy-, Acetyloxy-, Carboxy-, Ethoxycarbonyl-, Aminocarbonyl-, Methylaminocarbonyl-, Dimethylaminocarbonyl-, Cyano-, Amino-, Methylamino-, Dimethylamino-, Benzylamino-, Acetylamino-, Benzoylamino- und Amidinogruppen infrage.

n bedeutet vorzugsweise 0, 1 oder 2.

Bevorzugte Verbindungen der Formel I sind Verbindungen, in denen der Ring 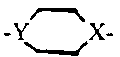 einen 1,4-Piperidinyl oder 1,4-Piperazenyl-Ring darstellt und die Symbole Z, A, W, n und R¹ die angegebene Bedeutung haben.

Weiterhin sind Verbindungen der Formel I bevorzugt, in denen W eine 〉CH- oder einen 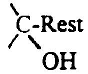 darstellt und die anderen Symbole die angegebene Bedeutung haben.

Insbesondere sind Verbindungen der Formel I bevorzugt, in denen A die Gruppe 〉NH; n die Zahlen 0, 1 oder 2; der Ring 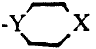 einen 1,4-Piperidinyl- oder 1,4-Piperazinyl-Ring und W einen 〉̶CH- oder 〉̶C-OH-Rest darstellen und Z und R¹ die angegebene Bedeutung haben.

Verbindungen der allgemeinen Formel I enthalten mindestens ein asymmetrisches Kohlenstoffatom, daher sind auch optisch aktive Verbindungen der allgemeinen Formel I Gegenstand der vorliegenden Anmeldung. Gegenstand der vorliegenden Anmeldung sind weiterhin Konformationsisomere von Verbindungen der allgemeinen Formel I, die gegebenenfalls auftreten können.

Verbindungen der allgemeinen Formel I werden nach an sich bekannten Verfahren durch Hydrolyse eines Esters der allgemeinen Formel II, 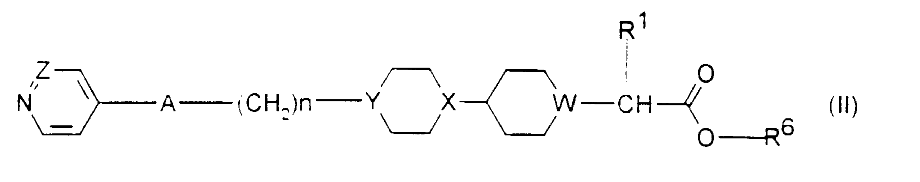 in der R¹, A, W, X, Y, Z und n die oben angegebenen Bedeutungen haben und R⁶ einen Methyl-, Ethyl-, tert.-Butyl- oder Benzylrest bedeutet, hergestellt.

Verbindungen der allgemeinen Formel II sind neu und werden nach an sich bekannten Verfahren hergestellt, vorzugsweise dadurch, daß man
a) für den Fall, daß W Stickstoff bedeutet, eine Verbindung der allgemeinen Formel III, 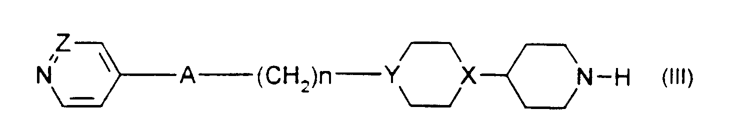 in der A, X, Y, Z und n die oben angegebenen Bedeutungen besitzen, mit einer Verbindung der allgemeinen Formel IV, 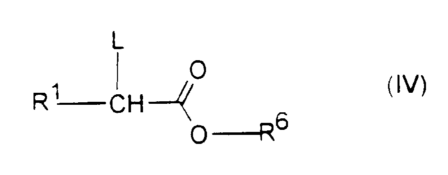 in der R¹ und R⁶ die oben angegebenen Bedeutungen haben und L eine Abgangsgruppe wie z.B. Hal oder O-SO₂-R⁷ bedeuten, wobei Hal, Chlorid, Bromid oder Jodid und R⁷ Methyl, Phenyl, p-Methylphenyl oder p-Nitrophenyl sein können. umsetzt,
b) für den Fall, daß W eine Gruppe CR⁵ und R⁵ eine Gruppe OR² bedeuten, ein Keton der allgemeinen Formel V, 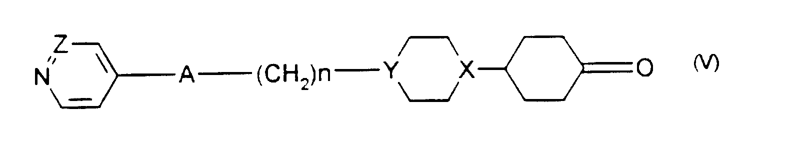 in der A, X, Y, Z und n die oben angegebenen Bedeutungen besitzen, mit einem Carbonsäureester der allgemeinen Formel VI, 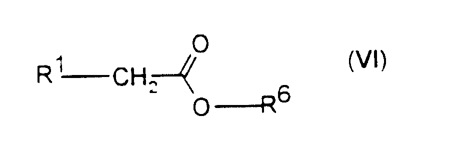 in der R¹ und R⁶ die oben angegebenen Bedeutungen haben zur Reaktion bringt und die Hydroxylgruppe des dabei entsprechenden 2-Hydroxyesters der allgemeinen Formel VII, 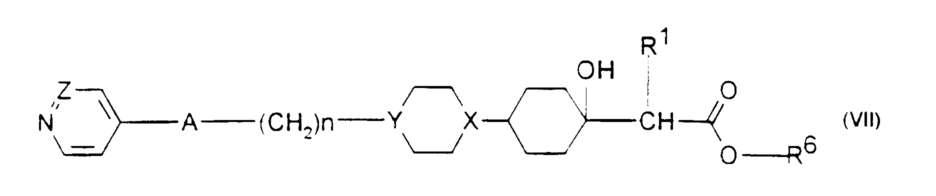 in der R¹, R⁶, X, Y, Z, A und n die oben angegebenen Bedeutungen besitzen, gegebenenfalls mit einem Alkylierungsmittel der allgemeinen Formel VIII,

   R²―L (VIII)

   in der R² und L die oben angegebenen Bedeutungen haben, alkyliert, oder
c) für den Fall, daß W die CH-Gruppe bedeutet, eine Verbindung der allgemeinen Formel VI mit einer Verbindung der allgemeinen Formel IX, 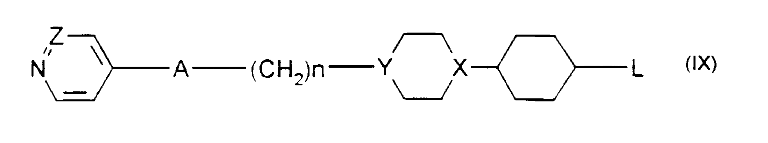 in der A, X, Y, Z, L und n die oben angegebenen Bedeutungen besitzen, alkyliert, oder
d) die olefinische Doppelbindung einer Verbindung der allgemeinen Formel X, 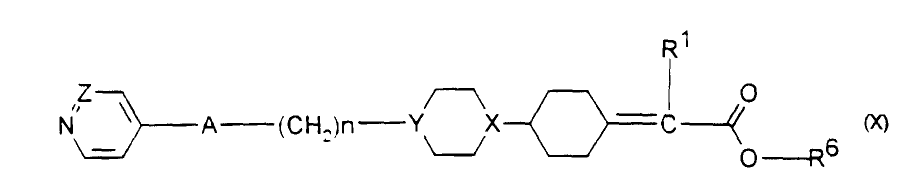 in der A, R¹, R⁶, X, Y, Z und n die oben angegebenen Bedeutungen besitzen, katalytisch hydriert.

Verbindungen der allgemeinen Formel III sind neu und werden nach an sich bekannten Verfahren hergestellt, vorzugsweise dadurch, daß man
1. für den Fall, daß X Stickstoff bedeutet, eine Verbindung der allgemeinen Formel XI, 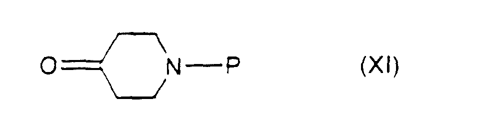 in der P die oben angegebenen Bedeutungen besitzt, einer reduktiven Aminierung mit einem Amin der allgemeinen Formel XII, 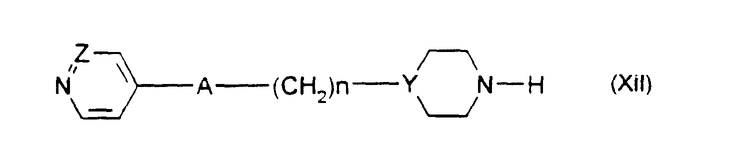 in der A, Y, Z und n die angegebenen Bedeutungen besitzen, unterwirft und anschließend aus dem entstandenen Produkt die Schutzgruppe P entfernt, oder
2. für den Fall, daß X = CH bedeutet, eine Verbindung der allgemeinen Formel XIII, 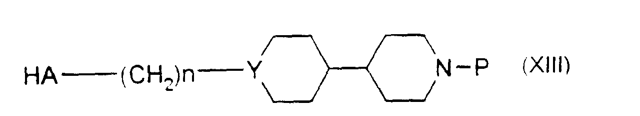 in der A, P, Y und n die oben angegebenen Bedeutungen besitzen, und A nicht der Valenzstrich sein darf, wenn n > 0 ist, mit einer Verbindung der allgemeinen Formel XIV, 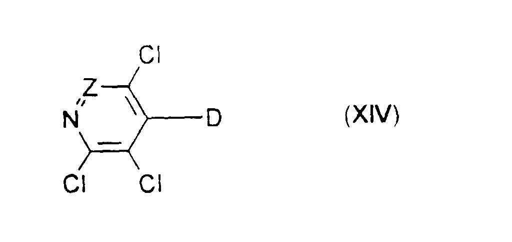 in der Z die oben angegebenen Bedeutungen besitzt oder Z = C-Cl bedeutet und D die Bedeutung von L hat oder die Nitrogruppe bedeutet zur Reaktion bringt und anschließend die Chloratome und die Schutzgruppe P aus dem Produkt entfernt.

Verbindungen der allgemeinen Formel IV werden so hergestellt, daß man für den Fall, daß L = Hal bedeutet, eine Verbindung der allgemeinen Formel VI, nach literaturbekannten Verfahren halogeniert, oder für den Fall, daß L in Formel IV die O-SO₂-R⁷-Gruppe bedeutet, die Hydroxylgruppe einer Verbindung der allgemeinen Formel XV, 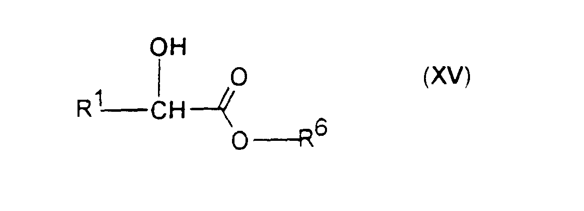 in der R¹ und R⁶ die oben angegebenen Bedeutungen besitzen, in den entsprechenden Sulfonsaureester überführt.

Verbindungen der allgemeinen Formel V werden in der Regel durch Spaltung der Ketalgruppe einer Verbindung der allgemeinen Formel XVI, 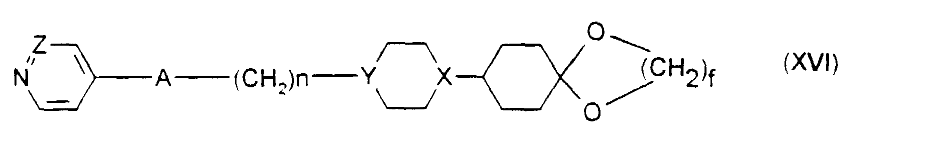 in der A, X, Y, Z und n die oben genannten Bedeutungen besitzen und f= 2.3 bedeutet, erhalten.

Verbindungen der allgemeinen Formel VIII sind, für den Fall, daß L = Hal bedeutet, käuflich erhältlich; für den Fall, daß L die O-SO₂-R⁷-Gruppe bedeutet, wird die Hydroxylgruppe käuflich erhältlicher Alkohole der allgemeinen Formel XVII,

R²―OH (XVII)

in der R² die oben angegebenen Bedeutungen besitzt, in den entsprechenden Sulfonsaureester überführt.

Verbindungen der allgemeinen Formel IX werden, für den Fall, daß L die OSO₂-R⁷-Gruppe bedeutet, dadurch hergestellt, daß man die Hydroxylgruppe einer Verbindung der allgemeinen Formel XVIII, 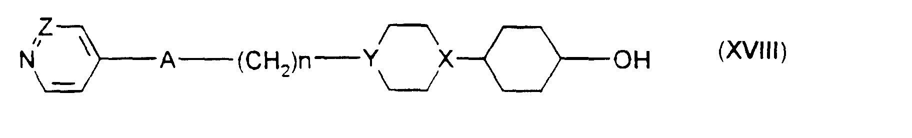 in der A, X, Y, Z und n die oben angegebenen Bedeutungen besitzen, in den entsprechenden Sulfonsaureester überführt; für den Fall, daß L = Hal bedeutet, wird die Hydroxylgruppe einer Verbindung der allgemeinen Formei XVIII nach literaturbekannten Verfahren durch Halogen nucleophil substituiert.

Verbindungen der allgemeinen Formel X sind neu und werden in an sich bekannter Weise dadurch hergestellt, daß man ein Keton der allgemeinen Formel V einer Wittig-Reaktion mit einem Phosphoran der allgemeinen Formel XIX, 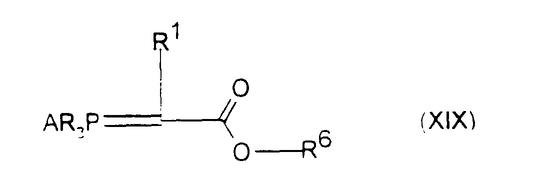 in der R¹ und R⁶ die oben angegebenen Bedeutungen besitzen und Ar ein Aryl im Sinne der oben angegebenen Definition für Aryl bedeutet, oder das Keton der Formel V einer Horner-Emmons Reaktion mit einem Phosphonoessigsäureester der allgemeinen Formel XX, 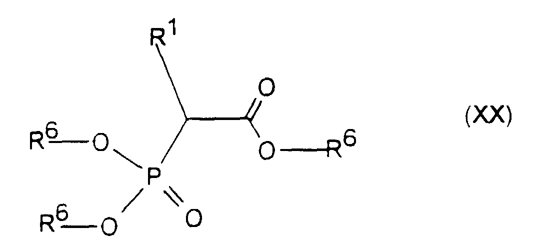 in der R¹ und R⁶ die oben angegebenen Bedeutungen besitzen, unterwirft.

Verbindungen der allgemeinen Formel XI werden so hergestellt, daß man das käufliche Piperidin-4-on entsprechend acyliert.

Verbindungen der allgemeinen Formel XII werden dadurch hergestellt, daß man
a) eine Verbindung der allgemeinen Formel XXI, 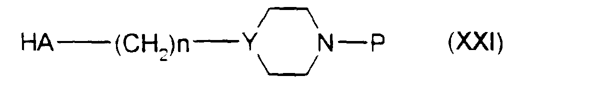 in der A, P, Y und n die oben angegebenen Bedeutungen besitzen und A nicht der Valenzstrich sein darf, wenn in der Verbindung XXI n > 0 bedeutet, mit einer Verbindung der allgemeinen Formel XIV zur Reaktion bringt und aus dem entstandenen Produkt die Cl-Atome und die Schutzgruppe P entfernt, oder
b) eine Verbindung der allgemeinen Formel XXII, 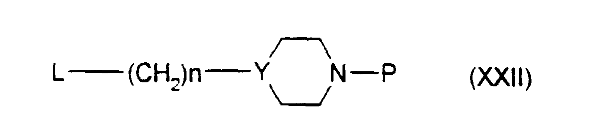 in der P, Y und L die oben angegebenen Bedeutungen besitzen und n > 0 sein muß, wenn Y = N bedeutet, mit einer Verbindung der allgemeinen Formel XXIII, 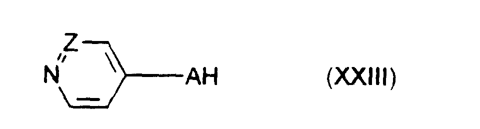 in der A und Z die oben angegebenen Bedeutungen mit der Ausnahme des Valenzstriches bedeuten, umsetzt.
c) für den Fall, daß in der Verbindung XII Y = N und n > 0 bedeuten, ein Piperazin der allgemeinen Formel XXIV, 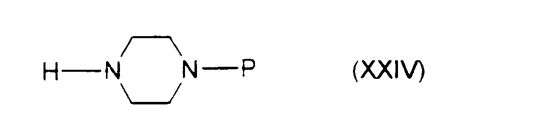 in der P die oben angegebenen Bedeutungen besitzt, mit einer Verbindung der allgemeinen Formel XXV, 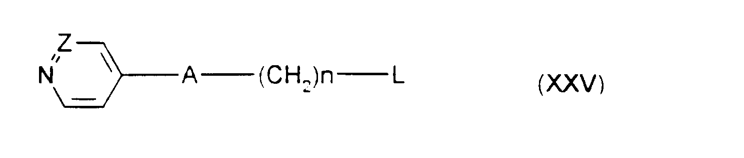 in der A, L, Z und n die oben angegebenen Bedeutungen besitzen, umsetzt.

Verbindungen der allgemeinen Formel XIII werden in an sich bekannter Weise dadurch hergestellt, daß man ein Keton der allgemeinen Formel XI mit einem Amin der allgemeinen Formel XXVI, 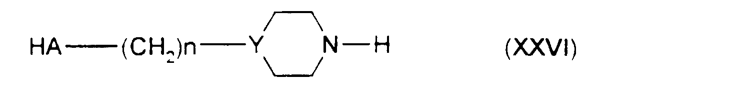 in der A, Y und n die oben angegebenen Bedeutungen besitzen, reduktiv aminiert.

Die Herstellung von Verbindungen der allgemeinen Formel XIV ist in den Patentschriften DE 4306506 und DE 4306873 beschrieben.

Verbindungen der allgemeinen Formel XV lassen sich nach Literaturverfahren durch Oxydation der entsprechenden Verbindungen der allgemeinen Formel VI erhalten.

Verbindungen der allgemeinen Formel XVI werden dadurch hergestellt, daß man,
1. für den Fall, daß in der Formel XVI X = N bedeutet, ein Keton der allgemeinen Formel XXVII, 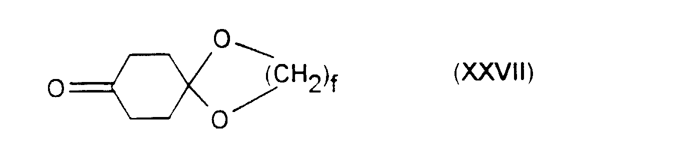 in der f die oben angegebenen Bedeutungen hat, mit einem Amin der allgemeinen Formel XII hydroaminiert, oder
2. für den Fall, daß in Verbindungen der Formel XVI, X = CH und Y = N bedeute, ein Amin der allgemeinen Formel XXVIII, 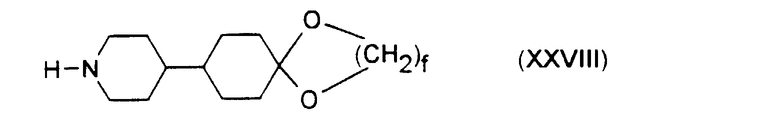 in der f die oben angegebenen Bedeutungen besitzt, mit einer Verbindung der allgemeinen Formel XXV zur Reaktion bringt,
   Alkohole der allgemeinen Formel XVII sind käuflich erhältlich.

Verbindungen der allgemeinen Formel XVIII werden dadurch hergestellt, daß man,
1. für den Fall, daß in Verbindungen der Formel XVIII X = N bedeutet, 4-Hydroxycyclohexanon der Formel XXIX, 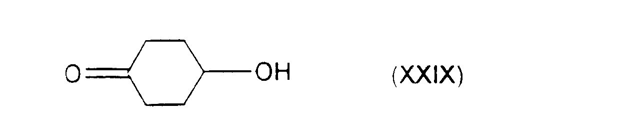 mit einen Amin der Formel XII reduktiv aminiert, oder
2. für den Fall, daß in Verbindungen der Formel XVIII X = CH und Y = N bedeuten, ein Amin der Formel XXX, 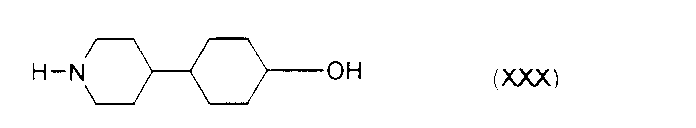 mit einer Verbindung der allgemeinen Formel XXV alkyliert.

Verbindungen der allgemeinen Formel XIX sind teilweise käuflich erwerblich (Aldrich-Chemie GmbH u. Co.KG) und werden in Spezialfällen nach bekannten Verfahren durch Umsetzung eines 2-Halogensäurederivats der allgemeinen Formel IV mit einem Triarylphosphin der allgemeinen Formel XXXI,

AR₃P (XXXI)

in der Ar die oben angegebenen Bedeutungen besitzt, erhalten.

Verbindungen der allgemeinen Formel XX sind teilweise käuflich erwerblich (Aldrich-Chemie GmbH u. Co.KG) und werden in Spezialfällen nach bekannten Verfahren durch die Arbuzov-Reaktion zwischen einem 2-Halogencarbonsäurederivat der Formel IV und einem Trialkylphosphit der allgemeinen Formel XXXII,

(OR⁶)₃P (XXXII)

in der R⁶ die oben angegebenen Bedeutungen besitzt, erhalten.

Verbindungen der allgemeinen Formel XXI werden dadurch hergestellt, daß man,
1. für den Fall, daß Y = N, n > 0 bedeuten, und A nicht der Valenzstnch sein darf eine Verbindung der allgemeinen Formel XXXIII,

   HA―(CH₂)n―L (XXXIII)

   in der A, L und n die oben angegebenen Bedeutungen besitzen, mit einem Piperazin-Derivat der Formel XXIV umsetzt, oder
2. für den Fall, daß Y = N und n = 0 bedeuten, handelt es sich entweder um Verbindungen der allgemeinen Formel XXIV oder um ein Hydrazin der allgemeinen Formel XXXIV bzw. ein Hydroxylamin der allgemeinen Formel XXXV, 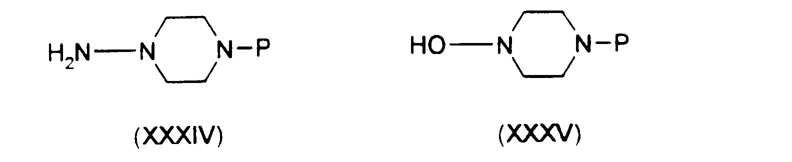 in der P die oben angegebenen Bedeutungen besitzen, die aus Verbindungen der allgemeinen Formel XXIV erhältlich sind.
3. für den Fall, daß Y = CH bedeutet, ein Carbonsäurederivat der allgemeinen Formel XXXVI, 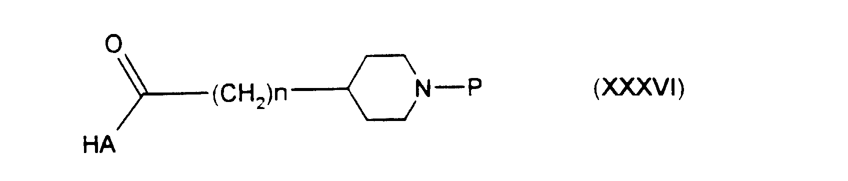 in der A, P und n die oben angegebenen Bedeutungen besitzen, reduziert.

Verbindungen der allgemeinen Formel XXII werden dadurch hergestellt, daß man,
1. für den Fall, daß Y = N und n > 0 bedeuten, ein Piperazin der Formel XXIV mit einer Verbindung der allgemeinen Formel XXXVII,

   L―(CH₂)n―L (XXXVII)

   in der L und n die oben angegebenen Bedeutungen besitzen, alkyliert, oder
2. für den Fall, daß Y = CH bedeutet, ein Alkohol der allgemeinen Formel XXXVIII, 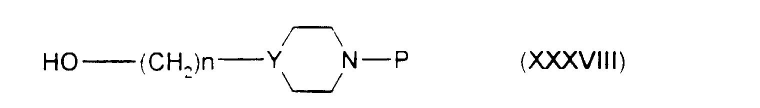 in der P und n die oben angegebenen Bedeutungen besitzen, in den entsprechenden Sulfonsäureester überführt, oder die Hydroxylgruppe gegen Halogen nach bekannten Verfahren ersetzt.

Verbindungen der allgemeinen Formel XXIII sind käuflich erwerblich.

Verbindungen der allgemeinen Formel XXIV sind käuflich erwerblich (Aldrich, GmbH u. Co.KG).

Verbindungen der allgemeinen Formel XXV werden nach bekannten Verfahren dadurch hergestellt, daß man ein Alkohol der allgemeinen Formel XXXIX, 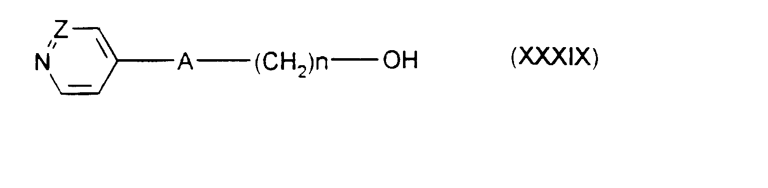 in der A, n und Z die oben angegebenen Bedeutungen besitzen, in den entsprechenden Sulfonsaureester überführt, oder die Hydroxylgruppe gegen Halogen ersetzt.

Verbindungen der Formel XXVI werden nach bekannten Verfahren aus Verbindungen der Formel XXI durch Hydrolyse der Schutzgruppe P hergestellt.

Verbindungen der allgemeinen Formel XXVII sind käuflich erwerblich (Aldrich-Chemie GmbH u. Co.KG).

Verbindungen der Formel XXVIII werden nach bekannten Verfahren durch Ketalisierung eines Ketons der Formel XXXX, 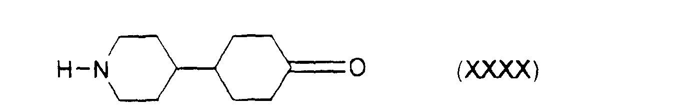 mit einem Diol der allgemeinen Formel XXXXI,

HO―(CH₂)_{f}―OH (XXXXI)

in der f die oben angegebenen Bedeutungen besitzt, erhalten.

4-Hydroxycyclohexanon der Formel XXIX wird dadurch erhalten, daß man die Ketalgruppe einer Verbindung der allgemeinen Formel XXXXII, 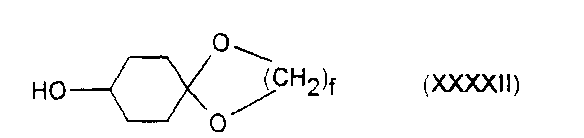 in der f die oben angegebenen Bedeutungen besitzt, entsprechend spaltet.

Die Herstellung von Verbindungen der allgemeinen Formel XXX ist in der Patentschrift EP 0537980 A 1 beschrieben.

Triarylphosphine der Formel XXXI und Trialkylphosphite der Formel XXXII sind käuflich erwerblich.

Verbindungen der allgemeinen Formel XXXIII sind für den Fall, daß L = Hal bedeutet käuflich erwerblich, und werden
für den Fall, daß L einen Sulfonsäurerest bedeutet, durch Überführung eines käuflich erwerblichen Alkohols der allgemeinen Formel XXXXIIII,

HA―(CH₂)n―OH (XXXXIII)

in der A und n die oben angegebenen Bedeutungen besitzen, in den Sulfonsäureester erhalten.

Verbindungen der allgemeinen Formel XXXIV werden durch Reduktion von N-Nitrosoverbindungen der allgemeinen Formel XXXXIV, 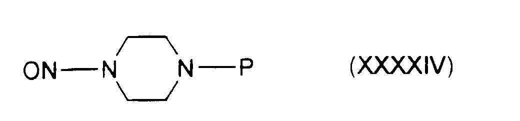 in der P die oben angegebenen Bedeutungen besitzt, hergestellt.

Verbindungen der allgemeinen Formel XXXV werden durch Oxidation von Verbindungen der allgemeinen Formel XXIV erhalten.

Verbindungen der allgemeinen Formel XXXVI werden nach bekannten Verfahren dadurch hergestellt, daß man eine Verbindung der allgemeinen Formel XXXXV, 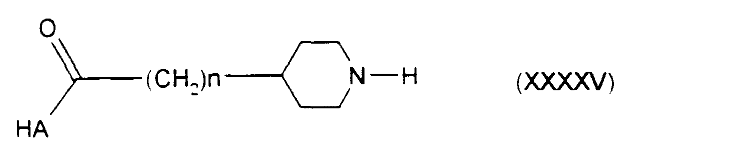 in der A und n die oben angegebenen Bedeutungen besitzen, entsprechend acyliert.

Verbindungen der allgemeinen Formel XXXVII sind für den Fall, daß L = Hal bedeutet, käuflich erwerblich, für den Fall, daß L einen Sulfonsäurerest bedeutet, werden käuflich erhältliche 1-Omegadiole entsprechend sulfoniert.

Verbindungen der allgemeinen Formel XXXVIII werden durch Reduktion von Verbindungen der allgemeinen Formel XXXVI, in der A Sauerstoff bedeutet, erhalten.

Verbindungen der allgemeinen Formel XXXIX werden dadurch hergestellt, daß man eine Verbindung der allgemeinen Formel XIV mit einer Verbindung der allgemeinen Formel XXXXIII umsetzt, und aus dem entstandenen Produkt die Chloratome entfernt.

Verbindungen der allgemeinen Formel XXXX werden durch Oxidation eines Alkohols der Formel XXX hergestellt.

Diole der allgemeinen Formel XXXXI sind käuflich erwerblich.

Verbindungen der allgemeinen Formel XXXXII werden durch Reduktion eines Ketons der allgemeinen Formel XXVII erhalten.

Verbindungen der allgemeinen Formel XXXXIV werden durch Nitrosierung von Verbindungen der allgemeinen Formel XXIV hergestellt.

Verbindungen der allgemeinen Formel XXXXV sind zum Teil käuflich erwerblich, oder in der Literatur beschriebenen (Ishihara, Chem. Pharm. Bull. 41, 529 (1993); Merck u. Co. EP 478362).

Die Hydrolyse eines Ester der allgemeinen Formel II zu der entsprechenden Carbonsaure der allgemeinen Formel I führt man nach ublichen Verfahren durch, in dem man einen Carbonsäureester der allgemeinen Formel II in Wasser oder in einem Gemisch aus Wasser, Tetrahydrofuran, Dioxan, Methanol oder Ethanol vorzugsweise in einem Wasser/Tetrahydrofurangemisch mit einem Hydroxid wie Natrium-, Kalium-, oder Lithiumhydroxid, vorzugsweise Natrium- oder Lithiumhydroxid, oder mit einer Säure wie Salzsäure, Schwefelsäure oder Trifluoressigsäure, vorzugsweise Trifluoressigsäure und bei Temperaturen zwischen Raumtemperatur und 80°C, vorzugsweise bei Raumtemperatur, behandelt.

Die Umsetzung einer Verbindung der allgemeinen Formel III mit einer Verbindune der Formel IV oder einer Verbindung der Formel VII mit einer Verbindung der Formel VIII oder einer Verbindung der Formel VI mit einer Verbindung der Formel IX oder einer Verbindung der Formel XXII mit einer Verbindung XXIII oder einer Verbindung der Formel XXIV mit einer Verbindung der Formel XV oder einer Verbindung der Formel XXVIII mit einer Verbindune der Formel XXV oder einer Verbindung der Formel XXX mit einer Verbindung der Formel XXV oder einer Verbindune der Formel XXIV mit einer Verbindung XXXIII oder einer Verbindung der Formel XXIV mit einer Verbindung der Formel XXXVII erfolgt in der Regel in einem aprotischen Lösungsmittel wie Toluol, Tetrahydrofuran, Diethylether oder Dimethylformamid, vorzugsweise Dimethylformamid oder Tetrahydrofuran unter Verwendung einer Base wie Kaliumhydrid, Natriumhydrid, Kaliumcarbonat oder Natriumhydrogencarbonat, vorzugsweise Natriumhydrid oder Kaliumcarbonat und bei Temperaturen zwischen Raumtemperaturen und 180°C, vorzugsweise bei 120°C.

Die Reaktion eines Ketons der allgemeinen Formel V mit einem Ester der allgemeinen Formel VI findet unter den Bedingungen der Aldolreaktion, in einem Lösungsmittel wie Methanol, Ethanol, Toluol, Tetrahydrofuran, Diethylether oder Dimethylformamid, vorzugsweise Tetrahydrofuran oder Dimethylformamid, unter Verwendung einer Base wie Natrium- oder Kaliummethylat oder -ethylat, Natriumhydrid, Kaliumhydrid, Lithiumdiisopropylamid, Kaliumhexamethyldisilazid, vorzugsweise Natriumhydrid oder Lithiumdiisopropylamid und bei Temperaturen zwischen -78°C und 90°C bevorzugt jedoch bei -78°C und Raumtemperatur statt.

Die katalytische Hydrierung der olefinischen Doppelbindung einer Verbindung der allgemeinen Formel X wird analog zu literaturbekannten Verfahren durchgeführt (A, Nose, Chem. Pharm. Bull. 38, 2097(1990); Tamura M. Bull. Chem. Soc. Jpn. 53,561 (1980); Liu H.-J., Synth. Commun. 15, 965 (1985); Chido N., J. Chem. soc. Chem. Commun. 994 (1990); Büchi G., J. Amer. Chem. Soc. 89, 6745 (1967); Ernst I., Coll. Czech. Chem. Comm. 24, 3341 (1959); Johnson w.S., J. Amer. chem. Soc. 79, 1995 (1957); Muchowski J. M., Can. J. Chem. 47, 857 (1969)).

Die reduktive Aminierung eines Ketons der Formel XI mit einem Amin der Formel XII oder eines Ketons der Formel XI mit einem Amin der Formel XXVI oder eines Ketons der Formel XXVII mit einem Amin der Formel XII oder eines Ketons der Formel XXIX mit einem Amin der Formel XII erfolgt nach literaturbekannten Verfahren durch Umsetzung der Keton- und Aminkomponente in einem Lösungsmittel wie Methanol oder Ethanol in Gegenwart eines Reduktionsmittels wie Natriumcyanoborhydrid oder Natriumacetatoborhydrid unter Zugabe einer Brönsted.- oder Lewissäure wie Salzsäure, Essigsäure, Titantetrachlorid oder Titantetraisopropylat und bei einer Temperatur zwischen 0° und 100°C vorzugsweise bei Raumtemperatur, oder in Gegenwart eines Hydrierkatalysators wie Platindioxid und einer Wasserstoffatomosphäre (Borch R. F., Org. Synth. Coll. Vol. 6, 499(1988); Heinzelman R. V. Z. Chem. 8, 270(1968); Mattson R. J., J. Org. Chem. 55, 2552 (1990); Barney C. L. Tetr. Letters 31, 5547 (1990); Hutchins R. O., J. Org. Chem. 46, 3571 (1981)).

Die Umsetzung einer Verbindung der allgemeinen Formel XIV mit einer Verbindung der Formel XXII oder mit einer Verbindung der Formel XXI oder mit einer Verbindung der Formel XXXXIII führt man in der Regel in einem Lösungsmittel wie Tetrahydrofuran oder 1,4-Dioxan, vorzugsweise 1,4-Dioxan in Gegenwart einer tertiären Stickstoffbase wie Triethylamin oder Morpholin, vorzugsweise Triethylamin und bei einer Temperatur zwischen 0°C und 100°C bevorzugt jedoch bei Raumtemperatur durch.

Die Chloratome chlorhaltiger Pyridin- bzw. Pyridazinderivate können bei Bedarf durch katalytische Hydrierung in Gegenwart eines Katalysators wie Platindioxid oder Palladium auf Kohle (5 - 10proz.) in einem Lösungsmittel wie Methanol, Ethanol oder Tetrahydrofuran, vorzugsweise Methanol und in einer Wasserstoffatmosphäre unter Zugabe einer Base wie Natriummethylat, Natriumethylat, Kaliumcarbonat oder Natriumhydrogencarbonat vorzugsweise Kaliumcarbonat, bei einer Temperatur zwischen - 10°C und 80°C, bevorzugt jedoch bei Raumtemperatur und bei einem Reaktordruck zwischen 1 atm und 20 atm vorzugsweise 5 atm entfernt werden.

Die Schutzgruppe P läßt sich bei Bedarf aus Verbindungen, die die Schutzgruppe P tragen und die in dieser Patentschrift beschrieben oder umfaßt sind, dadurch entfernen, daß man eine die Schutzgruppe P tragende Verbindung mit wäßrigen Mineralsäuren bzw. -basen, wie Salzsäure, Schwefelsäure oder Trifluoressigsäure bzw. Natron- oder Kalilauge behandelt oder diese einer katalytischen Hydrierung, wie z. B. mit Palladium/Kohle/Wasserstoff unterwirft.

Die Halogenierung einer Verbindung der allgemeinen Formel VI oder der Formel XVIII, oder der Formel XXXVIII oder der Formel XXXIX, erfolgt durch ihre Umsetzung mit molekularem Halogen (Chlor, Brom, Iod) vorzugsweise Brom ohne Lösungsmittel oder in einem inerten Lösungsmittel wie Methylenchlorid, Chloroform oder Tetrachlorkohlenstoff, vorzugsweise Tetrachlorkohlenstoff und unter Zusatz von rotem Phosphor. Phosphortrichlorid oder Phosphortribormid und bei einer Temperatur zwischen Raumtemperatur und 100°C, vorzugsweise bei 90°C (K. Stoh, Chem. Pharm. Bull. 34, 2078 (1986); H. J. Ziegler, Synthesis 1969, 39)). Weiter lassen sich Verbindungen der allgemeinen Formel VI dadurch halogenieren, daß man sie in einem aprotischen Lösungsmittel wie Tetrahydrofuran und bei niedriger Temperatur, bevorzugt bei -78°C mit einem Lithiumamid wie Lithiumdiisopropylamid metalliert und anschließend die in α-Stellung metallierte Verbindungen der allgemeinen Formel XVI mit Brom, Iod, Tetrachlorkohlenstoff oder Tetrabromkohlenstoff (M. Hesse. Helv. Chim. Acta 72, 847 (1989) R. T. Arnold, J. Org. Chem. 43, 3687 (1978)) bzw. mit N-Chlor- oder N-Bromsuccinimid (W. Oppolzer, Tetrahedron Lett. 26. 5037 (1985) umsetzt.

Die Überführung der Hydroxylgruppe einer Verbindung der allgemeinen Formel XV, XVII, XVIII, XXXVIII, XXXIX oder XXXXIII in einen Sulfonsäureester erfolgt nach üblichen Verfahren, wie z.B. durch die Kondensation mit einem Sulfonsäurechlorid, wie Methan-, Benzol, p-Toluol- oder p-Nitrobenzolsulfonsäurechlorid, vorzugsweise Methan- oder p-Toluolsulfonsäurechlorid, in einem inerten Lösungsmittel wie Methylenchlorid, Tetrahydrofuran oder Diethylether, vorzugsweise Methylenchlorid unter Verwendung einer Hilfsbase wie Trimethyl- oder Triethylamin oder Pyridin, vorzugsweise Triethylamin und bei einer Temperatur zwischen 0°C und Raumtemperatur.

Die Ketalspaltung eines Ketals der allgemeinen Formel XVI oder der Formel XXXXII bzw. die Ketalisierung eines Ketons der allgemeinen Formel XXXX mit einem Diol der Formel XXXXI werden nach Standardverfahren der organischen Chemie durchgeführt (ORGANIKUM; VEB Deutscher Verlag der Wissenschaften, Berlin 1977, Seite 486, 490).

Piperazine der allgemeinen Formel XXIV sind käuflich erwerblich (Aldrich-Chemie GmbH u. Co.KG).

Die Wittig Reaktion zwischen einem Keton der allgemeinen Formel V und einem Phosphoran der allgemeinen Formel XIX erfolgt nach bekannten Verfahren durch Rückflußerhitzen der Reaktanten in einem aprotischen Lösungsmittel wie Benzol, Toluol oder Xylol, vorzugsweise Toluol.

Die Horner-Emmons Reaktion zwischen einem Keton der allgemeinen Formel V und einem Phosphonoessigsäureester der allgemeinen Formel XX führt man in der Regel in einem Lösungsmittel wie Dimethylformamid, Tetrahydrofuran, Diethylether oder 1,4-Dioxan vorzugsweise Dimethylformamid oder Tetrahydrofuran unter Verwendung einer Base wie Natriumhydrid, Butyllithium, Lithiumdiisopropylamid oder Natriumhexamethyldisilazid, vorzugsweise Natriumhydrid oder Lithiumdiisopropylamid und bei einer Temperatur zwischen -78°C und 100°C bevorzugt jedoch bei -78°C oder Raumtemperatur durch. Die Oxidation einer Verbindung der allgemeinen Formel VI zu einer Verbindung der allgemeinen Formel XV führt man in der Regel in einem Lösungsmittel wie Tetrahydrofuran durch Zugabe einer Base wie Lithiumdiisopropylamid oder Lithium-N-Isopropyl-N-Cyclohexylamid unter Verwendung eines Oxidationsmittels wie einem Oxaziridin-Derivat, Molibdänperoxid oder Luftsauerstoff und bei Temperaturen zwischen -78°C und Raumtemperatur, vorzugsweise bei 50°C durch (C. Tamm, Tetrahedron Lett. 26, 203 (1085): F. A. Davis I. Org. Chem. 51, 2402 (1986); C. Wintoai Synth. Commun. 18, 2141 (1988)).

Die Umsetzung einer Verbindung der allgemeinen Formel IV mit einem Triarylphosphin der allgemeinen Formel XXXI wird analog zu literaturbekannten Verfahren durchgeführt (Buddras J., Angew. Chem. 80, 535 (1968); Bestmann H. J. Angew. Chem. 77, 620, 651 (1965); Wittig G. Ber. Deutsch. Chem. Ges. 88, 1654(1955).

Die Reaktion zwischen einer Verbindung der allgemeinen Formel IV mit einer Verbindung der allgemeinen Formel XXXII erfolgt in der Regel ohne Lösungsmittel bei Temperaturen zwischen Raumtemperatur und 150°C, vorzugsweise bei 130°C mit einer Reaktionszeit zwischen 30 min und 30 Stunden, vorzugsweise 18 Stunden.

Die Reduktion eines Carbonsäurederivats der alleemeinen Formel XXXVI wird in der Regel in einem Lösungsmittel wie Tetrahydrofuran oder Diethylether mit einem Reduktionsmittel wie Lithiumaluminiumhydrid und bei einer Reaktionstemperatur zwischen 0°C und Rückflußtemperatur des verwendeten Lösungsmittels, vorzugsweise bei 40°C durchgeführt.

Die Reduktion einer Nitrosoverbindung der allgemeinen Formel XXXXIV erfolgt nach bekannten Verfahren dadurch, daß man eine Verbindune der Formel XXXXIV in einem Lösungsmittel wie Wasser, Essigsäure, Ethanol, Tetrahydrofuran oder Diethylether, vorzugsweise Essigsäure der Tetrahydrofuran mit einem Reduktionsmittel wie elementares Zink, Lithiumaluminiumhydrid oder Natriumaluminiumhydrid, vorzugsweise elementares Zink oder Lithiumaluminiumhydrid und bei einer Temperatur zwischen Raumtemperatur und 120°C, bevorzugt jedoch bei 70°C umsetzt. Die Überführung einer Verbindung der allgemeinen Formel XXXXIV in einer Verbindung der Formel XXXIV kann auch hydrogenolytisch unter Verbindung eines Katalysators wie Palladium/Kohle stattfinden (Hatt, H. H., Org. Synth. Coll. Vol. 2, 211 (1943); Schüler F. W., J. Amer. Chem. Soc. 73, 4996 (1951); Schultz M. J. Prakt. Chem. 316, 347 (1974); Smith G.W., Ind. Eng. Chem. Prod. Res. Dev. 1, 117 (1962)).

Die Herstellung eines Hydroxylamins der allgemeinen Formel XXXV erfolgt nach bekannten Verfahren durch Oxidation eines Amins der allgemeinen Formel XXIV in einem Lösungsmittel wie Aceton, Methanol, Wasser oder Methylenchlorid, vorzugsweise Methanol, oder Methylenchlorid mit einem Oxidationsmittel wie Wasserstoffperoxid oder meta-Chlorperbenzoesäure unter Zusatz eines Katalysators wie Natriumwolframat und bei einer Temperatur zwischen 0°C und 60°C vorzugsweise bei Raumtemperatur (Gorrod J. W., Arch. Pharm. 319, 261 (1986); Sumitomo Chem. Ind. KK. JP 2212363).

Die Acylierung einer Verbindung der allgemeinen Formel XXXXV führt man in der Regel in einem Lösungsmittel wie Methylenchlorid, Dimethylformamid oder Pyridin, vorzugsweise Methylenchlorid oder Pyridin mit einem Acylierungsmittel wie Acetylchlorid, Di-tert.-Butyldicarbonat oder Benzyloxycarbonylchlorid unter Zusatz einer Hilfsbase wie Triethylamin oder 4-Dimethylaminopyridin und bei einer Temperatur zwischen -10°C und 50°C bevorzugt jedoch bei Raumtemperatur durch.

Die Oxidation eines Alkohols der allgemeinen Formel XXX zu einem Keton der allgemeinen Formel XXXX erfolgt nach bekannten Verfahren wie der Jones-Oxidation (Jones E. R. H., J. Chem. Soc. 36 (1946)), der Swern-Oxidation (Swern D., Tetrahedron 34, 1651 (1978), der Dess-Martin Oxidation (Dess D. B., Martin J. C., J. Org. Chem. 48, 4155 (1983) oder mit einem Brom-Urotropin-Komplex als Oxidationsmittel (Yavari I., J. Chem. Res. (S) 274 (1994).

Die Reduktion eines Ketons der allgemeinen Formel XXVII führt man in der Regel in einem Lösungsmittel wie Methanol, Ethanol, Tetrahydrofuran oder Diethylether vorzugsweise Methanol mit einem Reduktionsmittel wie Natriumborhydrid, Lithiumborhydrid, oder Lithiumaluminiumhydrid, vorzugsweise Natriumborhydrid und bei einer Temperatur zwischen -10°C und +30°C vorzugsweise bei Raumtemperatur durch.

Die Nitrosierung einer Verbindung der allgemeinen Formel XXIV zu einer Verbindung der Formel XXXXIV führt man in der Regel mit Natriumnitrit oder Isomethylnitrit in Wasser oder Ethanol unter Zusatz einer Säure wie Salzsäure oder Essigsäure und bei einer Temperatur zwischen -20°C und 80°C, vorzugsweise bei Raumtemperatur, durch.

Als pharmakologisch verträgliche Salze werden vor allem Alkalisalze, Ammoniumsalze, Trifluoracetate oder Hydrochloride verwendet, die man üblicher Weise z.B. durch Titration der Verbindungen mit anorganischen oder organischen Basen oder Säuren wie z.B. Natrium- oder Kaliumhydrogencarbonat, Natronlauge, Kalilauge, wäßrigem Ammoniak oder Amine wie z.B. Trimethyl- oder Triethylamin, Trifluoressigsäure oder Salzsäure herstellt. Die Salze werden in der Regel durch Umfällen aus Wasser/Aceton gereinigt.

Die erfindungsgemäßen neuen Substanzen der Formel I und ihre Salze konnen in flüssiger oder fester Form enteral oder parenteral appliziert werden. Hierbei kommen alle üblichen Applikationsformen infrage, beispielsweise Tabletten, Kapseln, Dragees, Sirupe, Lösungen, Suspension etc.. Als Injektionsmedium kommt vorzugsweise Wasser zur Anwendung, welches die bei Injektionslösungen üblichen Zusätze wie Stabilisierungsmittel, Lösungsvermittler und Puffer enthält.

Derartige Zusätze sind z.B. Tartrat- und Citrat-Puffer, Ethanol, Komplexbildner (wie Ethylendiamintetraessigsäure und deren nichttoxische Salze), hochmolekulare Polymere (wie flüssiges Polyethylenoxid) zur Viskositätsregelung. Flüssige Trägerstoffe für Injektionslosungen müssen steril sein und werden vorzugsweise in Ampullen abgefüllt. Feste Trägerstoffe sind z. B. Stärke, Lactose, Mannit, Methylcellulose, Talkum, hochdisperse Kieselsäuren, höhermolekulare Fettsäuren (wie Stearinsäure), Gelantine, Agar-Agar, Calciumphosphat, Magnesiumstearat, tierische und pflanzliche Fette, feste hochmolekulare Polymere (wie Polyethylenglykole); für orale Applikation geeignete Zubereitungen können gewünschtenfalls Geschmacks- und Süßstoffe enthalten.

Die Dosierung kann von verschiedenen Faktoren, wie applikationsweise, Spezies, Alter und/oder individuellem Zustand abhängen. Die tägliche zu verabreichenden Dosen liegen bei etwa 10-1000 mg/Mensch, vorzugsweise bei 100-500 mg/Mensch und können auf einmal oder mehrere Male verteilt eingenommen werden.

Bevorzugt im Sinne der vorliegenden Erfindung sind außer den in den Beispielen genannten Verbindungen und durch Kombination aller in den Ansprüchen genannten Bedeutungen der Substituenten ableitbaren Verbindungen die folgenden Pyndin- bzw. Pyridazinderivate:
2. {4-[4-(Pyridin-4-ylamino-methyl)-piperidin-1-yl]cyclohexyl}-essigsäure
3. (4-{4-[2-(Pyridin-4-ylamino)-ethyl]-piperidin-1-yl}-cyclohexyl)-essigsäure
4. (4-{4-[3-Pyridin-4-ylamino)-propyl]-piperidin-1-yl}-cyclohexyl)-essigsäure
5. (4-{4-[4-(Pyridin-4-ylamino)-butyl]-piperidin-1-yl}-cyclohexyl)-essigsäure
6. (4-{4-[4-(Pyridin-4-ylamino)-butyl]-piperazin-1-yl}-cyclohexyl)-essigsäure
7. (4-{1-[2-(Pyridin-4-ylamino)-ethyl]-piperidin-4-yl}-cyclohexyl)-essigsäure
8. (4-{1-[3-(Pyridin-4-ylamino)-propyl]-piperidin-4-yl}-cyclohexyl)-essigsäure
9. (4-{1-[4-(Pyridin-4-ylamino)-butyl]-piperidin-4-yl}-cyclohexyl)-essigsäure
10. {1-Hydroxy-4-[1-(Pyridin-4-yloxy)-piperidin-4-yl]-cyclohexyl}-essigsäure
11. (1-Hydroxy-4-{4-[2-(Pyridin-4-ylamino)-ethyl]-piperidin-1-yl}-cyclohexyl-essigsäure
12. (1-Hydroxy-4-{4-[3-(Pyridin-4-ylamino)-propyl]-piperidin-1-yl}-cyclohexyl)-essigsäure; Fp. 121-123 °C
13. (1-Hydroxy-4-{4-[4-(Pyridin-4-ylamino)-butyl]-piperidin)-1-yl}-cyclohexyl)-essigsäure
14. (1-Hydroxy-4-{4-[4-(Pyridin-4-ylamino)-butyl]-piperazin-1-yl}-cyclohexyl)-essigsäure
17. (1-Methoxy-4-{4-[2-(Pyridin-4-ylamino)-ethyl]-piperidin-1-yl}-cyclohexyl)-essigsäure
18. (1-Methoxy-4-{4-[2-(Pyridin-4-ylamino)-ethyl]-piperazin-1-yl}-cyclohexyl)-essigsäure
19. {4-[4-(Pyridin-4-yloxy)-piperazin-1-yl]-piperidin-1-yl}-cyclohexyl)-essigsäure
20. {1'-(Pyridin-4-yloxy)-[4,4']bipiperidinyl-1-yl}-essigsäure
21. {1'-[2-(Pyridin-4-ylamino)-ethyl]-[4,4']bipiperidinyl-1-yl}-essigsäure
22. (4-{4-[2-(Pyridin-4-ylamino)-ethyl]-piperazin-1-yl}-piperidin-1-yl)-essigsäure
37. Methoxy-(4-{4-[2-(Pyridin4-ylamino)-ethyl]-piperazin-1-yl}-cyclohexyl)-essigsäure
38. (1-Hydroxy-4-{4-[2-(Pyridin-4-ylamino)-ethyl]-piperazin-1-yl}-cyclohexyl)-methoxy-essigsäure
39. Methoxy-(4-{4-[3-(Pyridin-4-ylamino)-propyl]-piperazin-1-yl}-cyclohexyl)-essigsäure
40. (±)-(1-Hydroxy-4-{4-[4-(pyridin-4-ylamino)-butyl]-piperidin-1-yl}-cyclohexyl)-methoxy-essigsäure
42. (4-{4-[2-(Pyridazin-4-ylamino)-ethyl]-piperidin-1-yl}-cyclohexyl)-essigsäure
43. (4-{4-[2-(Pyridazin-4-ylamino)-ethyl]-piperazin-1-yl}-cyclohexyl)-essigsäure
44. (4-{4-[4-(Pyridazin-4-ylamino)-butyl]-piperazin-1-yl}-cyclohexyl)-essigsäure
45. (1-Hydroxy-4-{4-[2-(Pyridazin-4-ylamino)-ethyl]-piperidin-1-yl}-cyclohexyl)-essigsäure
47. Methoxy-(4-{4-[2-(Pyridazin-4-ylamino)-ethyl]-piperidin-1-yl}-cyclohexyl)-essigsäure
48. {4-[4-(Pyridin-4-yloxy)-piperidin-1-yl]-cyclohexyl}-essigsäure
49. {1-Hydroxy-4-[4-(Pyridin-4-yloxy)-piperidin-1-yl]-cyclohexyl}-methoxy-essigsäure
50. {4-[4-(Pyridin-4-yloxy)-piperazin-1-yl]-cyclohexyl}-essigsäure
52. {4-[1-(Pyridin-4-ylamino)-piperidin-4-yl]-cyclohexyl}-essigsäure
53. {1-Hydroxy-4-[1-(Pyridin-4-ylamino)-piperidin-4-yl]-cyclohexyl}-methoxy-essigsäure
54. {4-[4-(Pyridin-4-ylamino)-piperazin-1-yl]-cyclohexyl}-essigsäure
56. {4-[4-(Pyridazin-4-yloxy)-piperidin-1-yl]-cyclohexyl}-essigsäure
57. 4-[4-(Pyridazin-4-yloxy)-piperazin-1-yl]-cyclohexyl}-essigsäure
58. {1-Hydroxy-4-[4-(pyridazin-4-yloxy)-piperazin-1-yl]-cyclohexyl}-methoxy-essigsäure
59. {4-[1-(Pyridazin-4-ylamino)-piperidin-4-yl]-cyclohexyl}-essigsäure
60. {4-[4-(Pyridazin-4-ylamino)-piperazin-1-yl]-cyclohexyl}-essigsäure
62. (4-{3-[(Pyridin-4-ylamino)-propyl]-piperidin-1-yl}-cyclohexyl)-essigsäure; Fp. 79-81 °C

Die nachfolgenden Beispiele zeigen einige der Verfahrensvarianten, die zur Synthese der erfindungsgemäßen Verbindungen verwendet werden können. Sie sollten jedoch nicht eine Einschränkung des Erfindungsgegenstandes darstellen. Die Struktur der Verbindungen wurde durch ¹H, und gegebenenfalls durch ¹³C-NMR-Spektroskopie sowie durch Massenspektrosmetrie gesichert. Die Reinheit der Substanzen wurde mittels C, H, N, sowie dünnschichtchromatographisch bestimmt.

### Beispiel 1

### [4-[4-(Pyridin-4-ylamino)piperidin-1-yl]cyclohexyl]essigsäure

a) 115 g 4-Hydroxy-phenyl-essigsäureethylester werden in einer Mischung von 10 ml einmolarer Natriumethylatlösung und 350 ml Ethanol 72 Std. bei 160° C und 200 bar mit Raney-Nickel hydriert. Der Ansatz wird anschließend eingedampft und das zurückbleibende Öl im Hochvakuum destilliert. Man erhält 76.4 g 4-Hydroxycyclohexan-essigsäureethylester vom Siedepunkt 120 - 121° C/2 mmHg. DC (Laufmittel: Dichlormethan/Methanol 9:1). Rf= 0.8.
b) 20.6 g der unter a) erhaltenen Verbindung wurden mit 130 ml abs. Dichlormethan versetzt und anschließend 76 g Dess/Martin-Oxidationsmittel (Triacetoxyperiodinan, J. Am. Chem., Soc. 113, 7277, 1991) zugesetzt. Der Ansatz wurde unter gelegentlichem Kühlen 48 Std. bei Raumtemperatur gerührt. Anschließend wurde die organische Phase mit einer gesättigten Natriumhydrogencarbonatlösung versetzt und 1 Std. unter guter Durchmischung gerührt. Der ausgefallene Rückstand wurde abgesaugt, die organische Phase abgetrennt und mit Natriumhydrogensulfit- und Natriumthiosulfatlösung geschüttelt. Nach Trocknen und Eindampfen des Lösungsmittels erhält man 16.5 g 4-Oxocyclohexan-essigsäureethylester als Öl. DC (Laufmittel: Dichlormethan/Methanol 9:1). Rf = 0.9.
c) Zu 100 ml abs. Dioxan wurden 8 7 g 1-Benzyl-4-aminopiperidin, 4,6 g Triethylamin und 10 g 4-Nitro-2,3,5,6-tetrachlorpyridin gegeben und 5 Std. bei Raumtemperatur gerührt. Nach dem Abdestillieren des Dioxans wurde der Rückstand in Methylenchlorid aufgenommen, mit 2 normaler Salzsäure versetzt und 30 min kräftig gerührt. Der ausgefallene Niederschlag wurde abgetrennt, in wenig Wasser aufgenommen, mit 2 normalem Ammoniak alkalisch gestellt und die wäßrige Lösung mit Dichlormethan mehrmals extrahiert. Man erhält 13.9 g N-(1-Benzylpiperidin-4-yl)-4-amino-2,3,5,6-tetrachlorpyridin als Öl. DC (Laufmittel: Toluol/Dioxan/Wasser 9 : 4.5 : 0.5), Rf= 0.6.
d) 13.8 g der unter c) erhaltenen Verbindung wurden unter Zusatz von 39 g Kaliumcarbonat in einer Mischung von 100 ml Methanol und 100 ml Tetrahydrofuran mit 1.5 g Palladium auf Kohle (10 %) 18 Std. bei Raumtemperatur und 40 mbar hydriert. Anschließend wurde diese Suspension vom Katalysator abgesaugt und das Filtrat eingedampft. Das zurückbleibende Öl wurde anschließend in einer Mischung aus 11 ml Eisessig, 91 ml Methanol und 91 ml Tetrahydrofuran nochmals mit 1.8 g Palladium auf Kohle (10 %) 40 Std. bei Raumtemperatur und 4 bar hydriert. Nach Abtrennen des Katalysators wurde die Lösung eingedampft, der Rückstand mit verdünnter Salzsäure versetzt und die saure Lösung mit Dichlormethan extrahiert. Nach Abtrennen der organischen Phase wurde die wäßrige Lösung mit Ammoniak alkalisch gestellt und eingedampft. Der Rückstand wurde 2 Std. mit Dichlormethan ausgekocht, der ungelöste Niederschlag abgetrennt und die organische Phase getrocknet und eingedampft. Aus dem Rückstand erhält man nach Kristallisation aus Aceton4.2 g N-(Piperidin-4-yl)-4-aminopyridin DC (Laufmittel: Isopropanol/Butylacetat/ Wasser/konzentrierter Ammoniak 50:30:15:5), Rf = 0.2.
e) 0.8 g der unter b) erhaltenen Verbindung, 4-Oxocyclohexanessigsäureethylester, und 0.9 g der unter d) erhaltenen Verbindung werden in 25 ml Methanol unter Zusatz von 1.5 ml einmolarer ethanolischer Salzsaure und 0.28 g Natriumcyanoborhydrid 5 Tage bei Raumtemperatur gerührt. Das Methanol wird anschließend abgedampft, der Ruckstand mit Ether versetzt und die organische Phase mit Natriumhydrogencarbonat, und Natriumchloridlösung gewaschen. Nach Trocknen und Eindampfen der Etherlösung erhält man 1.6 g [4-[4-(Pyridin-4-ylamino)piperidin-1-yl]cyclohexyl]essig- säureethylester als Öl. DC (Laufmittel: Isopropanol/Butylacetat/Wasser/konzentrierter Ammoniak 50:30:15:5), Rf = 0.8.
f) 1.5 g der unter e) erhaltenen Verbindung wurden in 30 ml Methanol und 10 ml einmolarer NaOH-Lösung 30 min bei 50°C gerührt. Anschließend wurde das Methanol abgedampft und der Rückstand nach Versetzen mit Wasser zweimal mit Dichlormethan extrahiert. Nach Abtrennen der organischen Phase wurde die Wasserphase auf einen Ionenaustauscher (Dowex 50, saure Form) aufgegeben und nach Spülen mit Wasser wurde die gewünschte Substanz durch Elution mit Ammoniak/Wasser 1 : 3 vom Harz gewaschen. Nach Eindampfen der gewünschten Fraktionen und Durcharbeiten des Rückstandes mit Aceton konnte der erhaltene Rückstand mit Essigester kristallisiert werden. Man erhält 360 mg der Titelverbindung [4-[4-(Pyridin-4-ylamino)piperidin-1-yl]cyclohexyl]essigsäure. DC (Laufmittel: Isopropanol/Butylacetat/Wasser/konzentrierter Ammoniak 50:30:15:5), Rf = 0.2.

### Beispiel 2

### [4-[4-[2-(Pyridin-4-ylamino)ethyl]piperazin-1-yl]cyclohexyl]essigsäure

a) Zu einer Suspension von 8.6 g Lithiumaluminiumhydrid in 200 ml abs. Ether wird eine Lösung von 35.3 g 4-Benzylpiperazin-1-acetonitril in 300 ml abs. Ether langsam zugetropft und 5 Std. bei Raumtemperatur nachgerührt. Anschließend wird unter Eiskühlung überschüssiges Lithiumaluminiumhydrid mit 25 ml Wasser vorsichtig zersetzt. Der Niederschlag wird abgesaugt, mit Ether gewaschen und die Etherlösung eingedampft. Das zurückbleibende Öl wird im Hochvakuum destilliert. Man erhält 16 g 2-(4-Benzylpiperazin-1-yl)ethylamin als Öl mit einem Siedepunkt von 118 - 121°/0.01 mmHg.
b) Analog Beispiel 1 c) erhält man aus 2.61 g 4-Nitro-2,3,5,6-tetrachlornitropyridin, 20 ml abs. Dioxan, 2.6 g der im Beispiel 2 a) erhaltenen Verbindung und 1.2 g Triethylamin nach 3 Std. bei Raumtemperatur 3.9 g N-[(4-Benzylpiperazin-1-yl)ethyl]-4-amino-2,3,5,6-tetrachlorpyridin als Öl. DC (Laufmittel: Toluol/Dioxan/Wasser 90:20:0.2), Rf = 0.61.
c) Analog Beispiel 1 d) wurden 5 g der unter Beispiel 2 b) erhaltenen Verbindung mit Palladium auf Kohle (10 %) hydriert. Nach Abtrennen des Katalysators und Aufarbeitung erhält man 3.5 g N-[(Piperazin-1-yl)ethyl]-4-aminopyridin als Öl. DC (Laufmittel: Essigester/mit Ammoniak gesättigtes Methanol 85 : 15), Rf = 0.18.
d) Analog Beispiel 1 e) erhält man aus 2.1 g der im Beispiel c) erhaltenen Verbindung, 1.62 g der in Beispiel 1 b) erhaltenen Verbindung und 0.56 g Natriumcyanoborhydrid in 50 ml Methanol und 3 ml einer einmolaren ethanolischer Salzsäure nach 3 Tagen Rühren bei Raumtemperatur 1.6 g [4-[4-[2-(Pyridin-4-ylamino)ethyl]piperazin-1-yl]cyclohexyl]-essigsäureethylester als Öl. DC (Laufinittel: Isopropanyl/Butylacetat/Wasser/konzentrierter Ammoniak 50:30:15:5), Rf= 0.58.
e) Analog Beispiel 1 f) werden 0.75 g der in Beispiel 2 d) erhaltenen Verbindung in 15 ml Methanol und 5 ml einmolarer NaOH-Lösung innerhalb von 30 min bei 50° hydrolysiert. Nach Reinigung über Ionenaustauscher und Eindampfen des Filtrats wird der erhaltene Rückstand mehrmals mit Toluol abdestilliert. Der dann erhaltene klebrige Schaum kann mit Methanol durchgearbeitet und kristallisiert werden. Man erhält 350 mg [4-[4-[2-(Pyridin-4-ylamino)ethyl]piperazin-1-yl]cyclohexyl]essigsäure vom Schmelzpunkt 220° C.

### Beispiel 3

### [4-[4-[3-(Pyridin-4-ylamino)propyl]piperazin-1-yl]cyclohexyl]essigsaure

a) 44 g Benzylpiperazin, 200 ml abs. THF, 39.4 g 1-Chlor-3-brompropan und 76 g Triethylamin werden 8 Std. am Rückfluß erhitzt. Nach Abtrennen des Niederschlags wird das Tetrahydrofuran eingeengt, der Rückstand in 500 ml Ether gelöst, zweimal mit Kohle behandelt und anschließend das Lösungsmittel abdestilliert. Nach Destillation im Hochvakuum erhält man 1-Benzyl-4-(3-chlorpropyl)piperazin vom Siedepunkt 145 - 148° C/0.3 mmHg. 22.75 g dieses Destillats wurden in 200 ml Ethanol gelöst und mit 15 g Phthalimidkalium versetzt und 56 Std. am Rückfluß erhitzt. Anschließend wurde das Lösungsmittel eingeengt, der Rückstand in 150 ml Dichlormethan gelöst und die organische Phase dreimal mit 50 ml zweimolarer Natronlauge und dreimal mit 50 ml Wasser gewaschen. Die organische Phase wurde danach getrocknet und eingeengt. Man erhalt 30 g 2-[3-(4-Benzylpiperazin-1-yl)propyl]isoindol-1,3-dion als viskoses Öl. DC (Laufmittel: Heptan/Methylethylketon/Ammoniakatomosphäre 2:1), Rf= 0.32.
b) 29.04 g der unter Beispiel 3 a) erhaltenen Verbindung wurden mit 4.3 ml 98proz. Hydrazinhydrat in 150 ml Ethanol 3 Std. am Rückfluß erhitzt. Der ausgefallene Niederschlag wird abgesaugt und anschließend mit 100 ml zweimolarer Salzsäure unter leichter Erwärmung 15 min umgerührt. Die ungelösten Anteile werden abgesaugt und die salzsaure Lösung alkalisch gestellt und viermal mit 40 ml Dichlormethan ausgeschüttelt. Die ethanolische Reaktionslösung aus dem Ansatz wird ebenfalls eingeengt und mit der Dichlormethanlösung vereinigt. Nach Trocknen und Einengen wird der Rückstand im Hochvakuum destilliert. Man erhält 15.2 g 3-(4-Benzylpiperazin-1-yl)propylamin vom Siedepunkt 144 - 146° C/0 01 mmHg.
c) Analog Beispiel 1 c) wurden 26.1 g 4-Nitro-2,3,5,6-Tetrachlorpyridin, 28 g des unter 3 b) erhaltenen Amins, 12.14 g Triethylamin und 200 ml abs. Dioxan umgesetzt und aufgearbeitet. Man erhält 37 5 g N-[(4-Benzylpiperazin-1-yl)propyl]-4-amino-2,3,5,6-tetrachlorpyridin als Öl. DC (Laufmittel: Dichlormethan/Methylethylketon/Methanol/ Eisessig/Wasser 50:17:24:3:5), Rf = 0.85.
d) Analog Beispiel 1 d) wurden 36 g der vorgenannten Verbindung (Beispiel 2 c) an Palladium auf Kohle (10 %) hydriert und aufgearbeitet. Man erhält 10.2 g N-[3-(Piperazin-1-yl)propyl]4-aminopyridin als Öl. DC (Laufmittel: Dichlormethan/mit Ammoniak gesättigtes Methanol 80 : 20), Rf= 0 62.
e) Analog Beispiel 1 e) wurden 2.2 g der vorgenannten Verbindung (Beispiel 2 d), 1.62 g der in Beispiel 1 d) erhaltenen Verbindung, 0.56 g Natriumcyanoborhydrid, 3 ml 1 molare ethanolische Salzsäure und 50 ml Methanol 3 Tage bei Raumtemperatur gerührt und anschließend aufgearbeitet. Man erhält 2.6 g [4-[4-[3-(Pyridin-4-yl amino)propyl]-piperazin-1-yl]cyclohexyl]essigsäureethylester als Öl. DC (Laufmittel Isopropanol/ Butylacetat/Wasser/konzentrierter Ammoniak 50:30:15:5), Rf= 0.45.
f) Analog Beispiel 1 f) wurden 1.55 g der vorgenannten Verbindung (Beispiel 2 e) mit 30 ml Methanol und 10 ml einmolarer Natriumlauge verseift und aufgearbeitet. Nach Reinigung über den Ionenaustauscher wurde der verbleibende Rückstand mehrmals mit Toluol abdestilliert, anschließend in wenig Wasser gelöst, mit Essigester ausgeschüttelt und die Wasserphase zur Trockne eingedampft. Man erhält nach Trocknung über Phosphorpentoxid 0.61 g [4-[4-[3-(Pyridin-4-ylamino)propyl]-piperazin-1-yl]cyclohexyl]-essigsäure als Schaum. DC (Laufmittel: Isopropanol/Butylacetat/Wasser/konzentrierter Ammoniak 50:30:15:5), Rf= 0.23.

### Beispiel 5

### [1-Hydroxy-4-[4-(pyridin-4-ylamino)piperidin-1-yl]cyclohexyl]essigsäure

a) Analog Beispiel 1 e) wurden 1.8 g der in Beispiel 1 d) erhaltenen Verbindung, 2.0 g 1-Hydroxy-4-oxocyclohexan-1-essigsäure-tert.-butylester (Herstellung analog EP 537980), 0.56 g Natriumcyanoborhydrid, 3 ml einmolare ethanolische Salzsäure und 50 ml Methanol 3 Tage bei Raumtemperatur gerührt und anschließend aufgearbeitet. Nach säulenchromatographischer Reinigung an Kieselgel (Laufmittel: Essigester/ mit Ammoniak gesättigtes Methanol 85 : 15) erhält man nach Eindampfen der gewunschten Fraktionen 0.62 g [1-Hydroxy-4-[4-(pyridin-4-ylamino)piperidin-1-yl]cyclohexyl]essigsäure-tert.-butylester als weißen Schaum. DC (Laufmittel: Essigester/mit Ammoniak gesättigtes Methanol 85 : 15), Rf= 0.79.
b) 0.62 g der vorangegangen Verbindung (Beispiel 5 a) wurden mit 1 ml Wasser und 10 ml Trifluoressigsäure 20 min bei Raumtemperatur gerührt. Nach Abdampfen des Lösungsmittels wurde der Rückstand in 30 ml gesättigte Natriumhydrogencarbonlösung gelöst und analog Beispiel 1 f) über einen Ionenaustauscher gereinigt. Nach Eindampfen der gewünschten Fraktionen wurde der hellbeige Schaum durch Anreiben mit Diethylether kristallisiert. Man erhält 0.41 g [1-Hydroxy-4-[4-(pyridin-4-ylamino)piperidin-1-yl]cyclohexyl]essigsäure vom Schmelzpunkt 261° C.

### Beispiel 6

### [1-Hydroxy-4-[4-[2-(pyridin-4-ylamino)ethyl]piperazin-1-yl]cyclohexyl]essigsäure

a) Analog Beispiel 1 e) wurden 2.1 g der in Beispiel 2 c) erhalten Verbindung, 2.0 g 1-Hydroxy-4-oxocyclohexan-1-essigsäure-tert.-butylester (Herstellung analog EP 537980), 0.56 g Natriumcyanoborhydrid, 3 ml einmolare ethanolische Salzsäure und 50 ml Methanol 5 Tage bei Raumtemperatur gerührt und anschließend aufgearbeitet. Nach säulenchromatographischer Reinigung an Kieselgel (Laufmittel: Dichlormethan/mit Ammoniak gesättigtes Methanol 85 : 15) erhält man nach Eindampfen der gewünschten Fraktionen 1.96 g [1-Hydroxy-4-[4-[2-(pyridin-4-ylamino)ethyl]-piperazin-1-yl]cyclohexyl]essigsäure-tert.-butylester als weißen Schaum. DC (Laufmittel: Dichlormethan/mit Ammoniak gesättigtes Methanol 85 : 15), Rf= 0.54.
b) Analog Beispiel 5 b) wurden 1.9 g der vorangegangenen Verbindung (Beispiel 6 a) mit 3 ml Wasser und 30 ml Trifluoressigsäure hydrolysiert und aufgearbeitet. Nach Reinigung über den Ionenaustauscher und Eindampfen der gewünschten Fraktionen erhält man einen beigefarbenen Schaum der aus Ethanol umkristallisiert werden kann. Man erhält 1.2 g [1-Hydroxy-4-[4-[2-(Pyridin-4-ylamino)ethyl]piperazin-1-yl]cyclohexyl]essigsäure als weiße Kristalle vom Schmp. 238 - 240 °C.

### Beispiel 7

### [1-Hydroxy-4-[4-[3-(Pyridin-4-ylamino)propyl]piperazin-1-yl]cyclohexyl]essigsäure

a) Analog Beispiel 1 e) erhält man aus 2.2 g der in Beispiel 3 d) erhaltenen Verbindung, 2.0 g 1-Hydroxy-4-oxocyclohexan-1-essigsäure-tert.-butylester (Herstellung analog EP 537980), 0.56 g Natriumcyanoborhydrid, 3 ml einmolare ethanolische Salzsäure und 50 ml Methanol nach 5 Tagen rühren bei Raumtemperatur und anschließende Aufarbeitung einen weißen Schaum. Nach säulenchromatographischer Reinigung an Kieselgel (Laufmittel: Dichlormethan/mit Ammoniak gesättigtes Methanol 85:15) und Eindampfen der gewünschten Fraktionen 1.5 g [1-Hydroxy4-[4-[3-(pyridin-4-ylamino)propyl]piperazin-1-yl]cyclohexyl]essigsäure tert.-butylester als farbloses Harz. DC (Laufmittel: Dichlormethan/mit Ammoniak gesättigtes Methanol 85:15), Rf = 0.6.
b) Analog Beispiel 5 b) wurden 1.5 g der vorgenannten Verbindung (Beispiel 7 a) mit 1.5 ml Wasser und 15 ml Trifluoressigsäure hydrolysiert und anschließend aufgearbeitet. Nach Reinigung über den Ionenaustauscher und Eindampfen der gewünschten Fraktionen konnte der Rückstand aus Ethanol kristallisiert werden. Man erhält 750 ml [1-Hydroxy-4-[4-[3-(pyridin-4-ylamino)propyl]piperazin-1-yl]cyclohexyl]essigsäure als weiße Kristalle vom Schmp. 242°C.

### Beispiel 8

### [4-(Pyridin-4-ylaminomethyl)piperidin-1-yl]cyclohexyl]essigsäure

a) 1 g 4-Nitro-2,3,5,6-tetrachlorpyridin, 0.65 g 4-Aminomethylpiperidin und 20 ml absolutes Dioxan wurden 1 Stunde bei Raumtemperatur gerührt. Anschließend wurde das Dioxan abdestilliert, der Rückstand mit Wasser versetzt, vom Ungelösten abgetrennt und die wäßrige Phase mehrmals mit Dichlormethan extrahiert. Nach Trocknen und Eindamfpen des Lösungsmittels kann der Rückstand aus wenig Diethylether kristallisiert werden. Man erhält 0.3 g 4-(2,3,5,6-Tetrachlorpyridin-4-ylaminomethyl)pyridin als farbloserer Kristalle. DC (Laufmittel: Toluol/Dioxan/Wasser 90:20:0.2), Rf= 0.8.
b) 47 g der vorgenannten Verbindung (Beispiel 9 a) wurden in 400 ml Methanol und 400 ml Tetrahydrofuran nach Zugabe von 164 g Kaliumcarbonat an 5 g Palladium auf Kohle (10 %) innerhalb von 18 Stunden bei Raumtemperatur und 40 mbar hydriert. Nach Abtrennung des Katalysators wurde das Lösungsmittels eingedampft, der verbleibende Rückstand in 150 ml Ethanol gelöst und durch Zugabe von etherischer Salzsäure ein Niederschlag ausgefällt. Das Kristallisat wurde abgesaugt, in 100 ml Wasser gelöst, mit konz. Ammoniak die Lösung alkalisch gestellt und anschließend mehrmals mit Dichlormethan ausgeschüttelt. Nach Trocknen und Eindampfen des Lösungsmittels erhält man 9 g 4-(Pyridin-4-ylaminomethyl)piperidin als Öl. DC (Laufmittel: Dichchlormethan/mit Ammoniak gesättigtes Methanol 85:15), Rf = 0.21.
c) Analog Beispiel 1 e) wurden 1.95 g der in Beispiel 9 b) erhaltenen Verbindung, 1.62 g der in Beispiel 1 d) erhaltenen Verbindung, 0.56 g Natriumcyanoborhydrid, 3 ml einmolare ethanolische Salzsäure und 50 ml Methanol 5 Tage bei Raumtemperatur gerührt und anschließend aufgearbeitet. Nach säulenchromatographischer Reinigung an Kieselgel (Laufmittel: Dichlormethan/mit Ammoniak gesättigtes Methanol 85:15) erhält man nach Eindampfen der gewünschten Fraktionen 0.5 g [4-[4-(Pyridin-4-ylaminomethyl)piperidin-1-yl)cyclohexyl]essigsäureethylester als gelbliches Harz. DC (Laufmittel: Dichlormethan/mit Ammoniak gesättigtes Methanol 85:15), RF = 0.58.
d) Analog Beispiel 1 f) wurde 1 g der vorgenannten Verbindung (Beispiel 9 c) mit 30 ml Methanol und 10 ml zweimolarer Natronlauge hydrolysiert und anschließend aufgearbeitet. Nach Reinigung über den Ionenaustauscher wurde der verbleibende Rückstand mehrmals mit Toluol abdestilliert und der Rückstand aus Ethanol kristallisiert. Man erhält 0.38 g [4-[4-(Pyridin-4-ylaminomethyl)piperidin-1-yl]cyclohexyl]essigsäure vom Schmp. 260°C.

### Beispiel 9

### [1-Hydroxy-4-[4-(pyridin-4-ylaminomethyl)piperidin-1-yl]cyclohexyl]essigsäure

a) Analog Beispiel 1 e) wurden 1.95 g der in Beispiel 9 b) erhaltenen Verbindung, 2.0 g 1-Hydroxy-4-oxocyclohexan-1-essigsäure-tert.-butylester (Herstellung analog EP 537980), 0.56 g Natriumcycanoborhydrid, 3 ml einmolare ethanolische Salzsäure und 50 ml Methanol 5 Tage bei Raumtemperatur gelöst und anschließend aufgearbeitet. Nach säulenchromatographischer Reinigung des Rohproduktes an Kieselgel (Laufmittel: Essigester/mit Ammoniak gesättigtes Methanol 85:15) und Eindampfen der gewünschten Fraktionen erhält man 0.55 g [1-Hydroxy-4-[4-(pyridin-4-ylaminomethyl)piperidin-1-yl]cyclohexyl]essigsäure-tert.-butylester als weißen Schaum. DC (Laufmittel: Essigester/mit Ammoniak gesättigtes Methanol 85:15), Rf = 0.48.
b) Analog Beispiel 5 b) wurden 0.51 g der vorgenannten Verbindung (Beispiel 10 a) mit 1 ml Wasser und 10 ml Trifluoressigsäure verseift und anschließend aufgearbeitet. Nach Reinigung über den Ionenaustauscher und Eindampfen der gewünschten Fraktionen konnte der weiße Schaum aus Ethanol kristallisiert werden. Man erhält 0.36 g [1-Hydroxy-4-[4-(pyridin-4-ylaminomethyl)piperidin-1-yl)cyclohexyl]- essigsäure als weiße Kristalle vom Schmp. 266°C.

### Pharmakologischer Test

### Assay

Microtiterplatten werden über Nacht mit 2 µg/ml isoliertem aktiviertem GpIIb/IIIa-Rezeptor beschichtet. Nach der Entfernung von ungebundenem Rezeptor durch mehrfaches Waschen wir die Oberfläche der Platten mit 1 % Kasein blockiert und nochmals gewaschen. Die Testsubstanz wird in den erforderlichen Konzentrationen zugefügt, und die Platten werden 10 Minuten unter Schütteln inkubiert. Der naturgemäße Ligand des gpIIb/IIIa-Rezeptors, Fibrinogen, wird hinzugefügt. Nach einer Stunde Inkubation wird das ungebundene Fibrinogen durch mehrfaches Waschen entfernt und das gebundene Fibrinogen wird durch einen Peroxidase-konjugierten, antifibrinogen monoklonaren Antikörper bestimmt durch Messung der optischen Dichte bei 405 nm in einem ELISA-Gerät. Die Inhibierung einer Fibrinogen-GpIIb/IIIa-Interaktion resultiert in einer niedrigen optischen Dichte. Ein IC₅₀ wird errechnet basierend auf einer Konzentrations-Wirkungs-Kurve.

### Literatur

Der GpIIb/IIIa-Finbrinogen-Elisa ist eine Modifikation von Assays, die in der folgenden Literatur beschrieben wird:

Nachman, R.L. & Leung, L.L.K. (1982): Complex formation of platelet membrane glycoproteins Iib and IIIa with fibrinogen. J. Clin. Invest. **69**:263-269: Wright, P.S. et al. (1993): An echistatin C-terminal peptide activated GpIIbIIIa binding to fibrinogen, fibronectin, vitronectin and collagen type I and type IV. Biochem. J. **293**:263-267.

### Pharmakologische Daten

| **Beispiel** | **IC**_{**50**} **(µMol/l)** |
|---|---|
| 5 | 0.14 |
| 12 (Seite 28) | 0.20 |
| 62 (Seite 32) | 0.30 |

### Vergleichsversuche

Als Vergleichssubstanz wurde die Verbindung cis-1-Hydroxy-4-[4-(4-pyridyl)-piperazin-1-yl]-cyclohexyl-essigsäure hergestellt, die in dem Patent WO 94/22835 als Beispiel Nr. 102 enthalten ist. Diese Verbindung besitzt im obigen Assay einen IC₅₀-Wert von **2.50** µMol/l!

## Patentansprüche

1. Verbindungen der Formel I 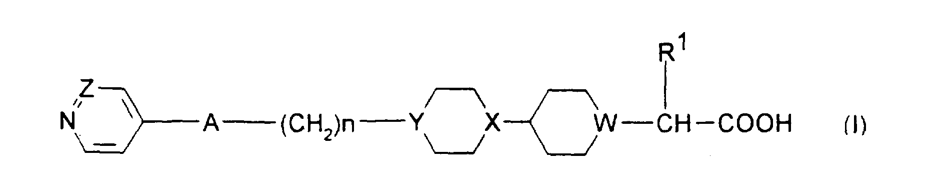 in der
R¹ Wasserstoff, C₁-C₆-Alkyl, C₃-C₆-Alkenyl, Cycloalkyl, Cycloalkenyl, ein gegebenenfalls substituiertes mono- oder bicyclisches Aryl, ein gegebenenfalls substituiertes Hetaryl, ein gegebenenfalls substituiertes Arylalkyl oder eine der Gruppen
-OR², -NR³R⁴
bedeutet,
W Stickstoff oder -CR⁵ bedeutet,
X, Y, Z unabhängig voneinander Stickstoff oder die Gruppe 〉̶CH bedeuten, und für den Fall, daß W die Gruppe 〉̶CR⁵ und X die Gruppe 〉̶CH bedeuten, Y nicht die 〉̶CH-Gruppe sein darf,
A Sauerstoff, 〉NR² oder 〉N-P bedeutet,
n 0 - 5 bedeutet,
P eine Schutzgruppe für Amine wie Acetyl, tert.-Butyloxycarbonyl oder Benzyloxycarbonyl bedeutet,
R² Wasserstoff, C₁-C₆-Alkyl oder Arylalkyl bedeutet,
R³, R⁴ unabhängig voneinander Wasserstoff, oder C₁-C₆-Alkyl bedeuten, oder zusammen mit dem Stickstoffatom, an das sie gebunden sind, einen fünf- bis sechsgliedrigen heterocyclischen Ring bilden,
R⁵ Wasserstoff oder eine Gruppe -OR² bedeutet,
und deren optische Isomere, sowie deren pharmakologisch unbedenkliche Salze.

2. Verfahren zur Herstellung von Verbindungen der Formel I 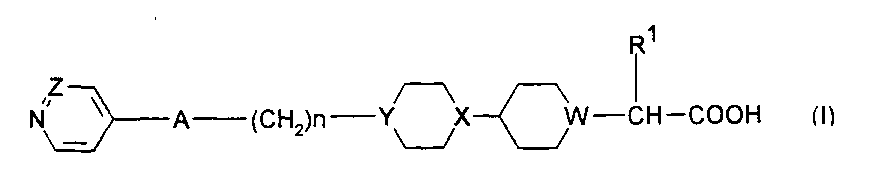 in der
R¹ Wasserstoff, C₁-C₆-Alkyl, C₃-C₆-Alkenyl, Cycloalkyl, Cycloalkenyl, ein gegebenenfalls substituiertes mono- oder bicyclisches Aryl, ein gegebenenfalls substituiertes Hetaryl, ein gegebenenfalls substituiertes Arylalkyl oder eine der Gruppen
-OR², -NR³R⁴
bedeutet,
W Stickstoff oder 〉̶CR⁵ bedeutet,
X, Y, Z unabhängig voneinander Stickstoff oder die Gruppe 〉̶CH bedeuten, und für den Fall, daß W die Gruppe 〉̶CR⁵ und X die Gruppe 〉̶CH bedeuten, Y nicht die 〉̶CH-Gruppe sein darf,
A Sauerstoff, 〉NR² oder 〉N-P bedeutet,
n 0 - 5 bedeutet,
P eine Schutzgruppe für Amine wie Acetyl, tert.-Butyloxycarbonyl oder Benzyloxycarbonyl bedeutet,
R² Wasserstoff, C₁-C₆-Alkyl oder Arylalkyl bedeutet,
R³, R⁴ unabhängig voneinander Wasserstoff, oder C₁-C₆-Alkyl bedeuten, oder zusammen mit dem Stickstoffatom, an das sie gebunden sind, einen fünf- bis sechsgliedrigen heterocyclischen Ring bilden,
R⁵ Wasserstoff oder eine Gruppe -OR² bedeutet,
und deren optische Isomere, sowie deren pharmakologisch unbedenkliche Salze,
dadurch gekennzeichnet, daß man in an sich bekannter Weise eine Verbindung der Formel II 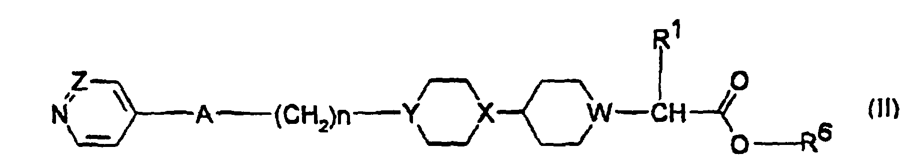 in der R¹, A, W, X, Y, Z und n die oben angegebene Bedeutung haben und R⁶ einen Methyl-, Ethyl-, ter.-Butyl- oder Benzylrest bedeutet, hydrolisiert,
und die erhaltenen Verbindungen gegebenenfalls in ihre optischen Isomere überführt und gewünschtenfalls erhaltene Verbindungen der Formel I in pharmakologisch unbedenkliche Salze überführt.

3. Arzneimittel, enthaltend mindestens eine Verbindung der Formel I gemäß Anspruch 1 nben üblichen Träger- und Hilfsstoffen.

4. Verwendung von Verbindungen der Formel I gemäß Anspruch 1 zur Herstellung von Arzneimitteln zur Behandlung von Krankheiten, die auf thromboembolische Ereignisse zurückzuführen sind.

## Claims

1. Compounds of formula I 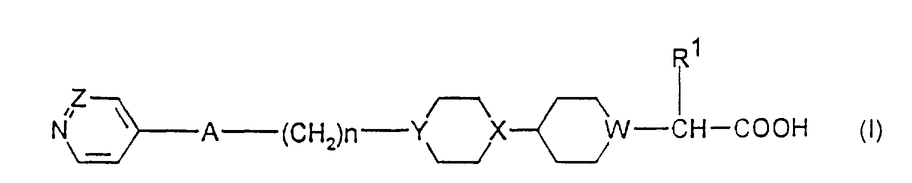 in which
R¹ denotes hydrogen, C₁-C₆ alkyl, C₃-C₆ alkenyl, cycloalkyl, cycloalkenyl, an optionally substituted monocyclic or bicyclic aryl, an optionally substituted hetaryl, an optionally substituted arylalkyl or one of the groups
-OR², -NR³R⁴
W denotes nitrogen or -CR⁵
X,Y,Z independently of one another denote nitrogen or the group -CH and in the case that W denotes the group -CR⁵ and X denotes the group -CH, Y cannot be the -CH group
A denotes oxygen, NR² or N-P
n denotes 0-5
P denotes a protecting group for amines such as acetyl, tert.-butyloxycarbonyl or benzyloxycarbonyl
R² denotes hydrogen, C₁-C₆ alkyl or arylalkyl
R³,R⁴ independently of one another denote hydrogen or C₁-C₆ alkyl or together with the nitrogen atom to which they are bound form a five or six-membered heterocyclic ring
R⁵ denotes hydrogen or a group -OR²
and optical isomers and pharmacologically acceptable salts thereof.

2. Process for the production of compounds of formula I 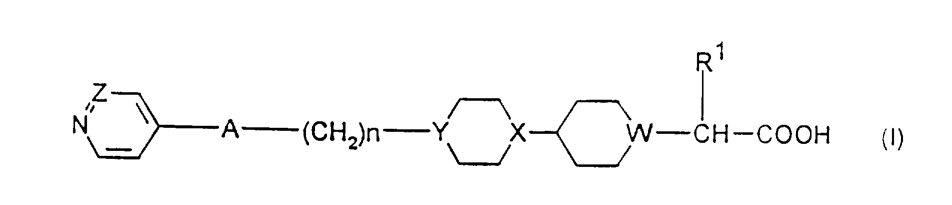 in which
R¹ denotes hydrogen, C₁-C₆ alkyl, C₃-C₆ alkenyl, cycloalkyl, cycloalkenyl, an optionally substituted monocyclic or bicyclic aryl, an optionally substituted hetaryl, an optionally substituted arylalkyl or one of the groups
-OR², -NR³R⁴
W denotes nitrogen or -CR⁵
X,Y,Z independently of one another denote nitrogen or the group -CH and in the case that W denotes the group -CR⁵ and X denotes the group -CH, Y cannot be the -CH group
A denotes oxygen, NR² or N-P
n denotes 0-5
P denotes a protecting group for amines such as acetyl, tert.-butyloxycarbonyl or benzyloxycarbonyl
R² denotes hydrogen, C₁-C₆ alkyl or arylalkyl
R³,R⁴ independently of one another denote hydrogen or C₁-C₆ alkyl or together with the nitrogen atom to which they are bound form a five or six-membered heterocyclic ring
R⁵ denotes hydrogen or a group -OR²
and optical isomers and pharmacologically acceptable salts thereof,
wherein, a compound of formula II 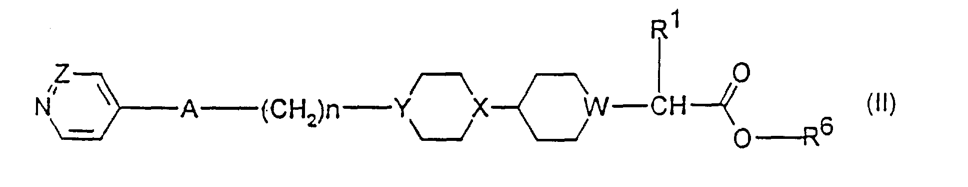 in which R¹, A, W, X, Y, Z and n have the meaning stated above and R⁶ denotes a methyl, ethyl, tert.-butyl or benzyl residue is hydrolyzed in a well-known manner
and the compounds obtained are optionally converted into their optical isomers and if desired compounds of formula I which are obtained are converted into pharmacologically acceptable salts.

3. Pharmaceutical preparations containing at least one compound of formula I as claimed in claim 1 in addition to conventional carriers and auxiliary substances.

4. Use of compounds of formula I as claimed in claim 1 to produce pharmaceutical preparations for the treatment of diseases which are a result of thromboembolic events.

## Revendications

1. Composés de formule I 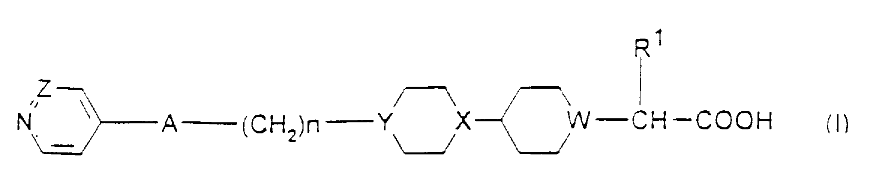 dans laquelle
R¹ représente un atome d'hydrogène, un groupe alkyle en C₁-C₆, alcényle en C₃-C₆, cycloalkyle, cycloalcényle. aryle mono- ou bicyclique éventuellement substitué, hétéroaryle éventuellement substitué, arylalkyle éventuellement substitué ou un groupe
-OR², -NR³R⁴
W représente un atome d'azote ou un groupe 〉̶CR⁵,
X,Y,Z représentent indépendamment un atome d'azote ou le groupe 〉̶CH, et si W représente le groupe 〉̶CR⁵ et X le groupe 〉̶CH, Y ne doit pas représenter le groupe 〉̶CH,
A représente un atome d'oxygène. un groupe >NR² ou >N-P,
n vaut 0-5,
P représente un groupe amino-protecteur tel qu'un groupe acétyle, tert.-butoxycarbonyle ou benzyloxycarbonyle,
R² représente un atome d'hydrogène, un groupe alkyle en C₁-C₆ ou arylalkyle,
R³, R⁴ représentent indépendamment un atome d'hydrogène ou un groupe alkyle en C₁-C₆, ou forment, avec l'atome d'azote auquel ils sont liés, un hétérocycle à cinq ou six maillons,
R⁵ représente un atome d'hydrogène ou un groupe -OR²,
et leurs isomères optiques ainsi que leurs sels pharmacologiquement acceptables.

2. Procédé de préparation de composés de formule I 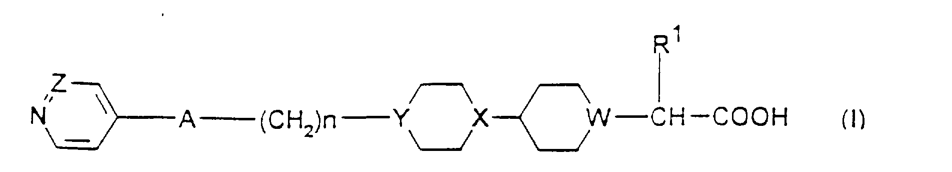 dans laquelle
R¹ représente un atome d'hydrogène, un groupe alkyle en C₁-C₆, alcényle en C₃-C₆, cycloalkyle, cycloalcényle. aryle mono- ou bicyclique éventuellement substitué, hétéroaryle éventuellement substitué, arylalkyle éventuellement substitué ou un groupe
-OR², -NR³R⁴
W représente un atome d'azote ou un groupe 〉̶CR⁵,
X,Y,Z représentent indépendamment un atome d'azote ou le groupe 〉̶CH, et si W représente le groupe 〉̶CR⁵ et X le groupe 〉̶CH, Y ne doit pas représenter le groupe 〉̶CH,
A représente un atome d'oxygène, un groupe >NR² ou >N-P,
n vaut 0-5,
P représente un groupe amino-protecteur tel qu'un groupe acétyle, tert.-butoxycarbonyle ou benzyloxycarbonyle,
R² représente un atome d'hydrogène, un groupe alkyle en C₁-C₆ ou arylalkyle,
R³, R⁴ représentent indépendamment un atome d'hydrogène ou un groupe alkyle en C₁-C₆, ou forment, avec l'atome d'azote auquel ils sont liés, un hétérocycle à cinq ou six maillons,
R⁵ représente un atome d'hydrogène ou un groupe -OR²,
et leurs isomères optiques ainsi que leurs sels pharmacologiquement acceptables,
caractérisé par le fait que l'on hydrolyse de manière connue en soi un composé de formule II 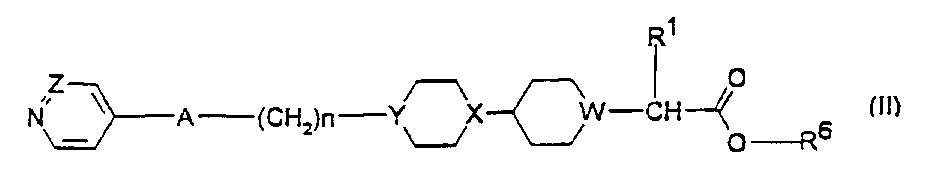 dans laquelle R¹, A, W, X, Y, Z et n ont les significations indiquées et R⁶ représente un groupe méthyle, éthyle, tert.-butyle ou benzyle,
et on transforme éventuellement les composés obtenus en leurs isomères optiques et les composés de formule I obtenus, éventuellement en leurs sels pharmacologiquement acceptables.

3. Médicament, contenant au moins un composé de formule I selon la revendication 1, en plus des véhicules et adjuvants usuels.

4. Utilisation de composés de formule I selon la revendication 1, pour la préparation de médicaments destinés au traitement de maladies d'origine thrombo-embolique.
